# NEUE EUROPÄISCHE PATENTSCHRIFT

(11) **EP 1 776 460 B2**
(45) Veröffentlichungstag und Bekanntmachung des Hinweises auf die Entscheidung über den Einspruch: **25.12.2013**
(45) Hinweis auf die Patenterteilung: 30.12.2009
(21) Anmeldenummer: 05768791.5
(22) Anmeldetag: 03.08.2005
(51) Int. Cl.: C12N 15/63

(54) **VERFAHREN ZUR MODULATION DER GENEXPRESSION DURCH ÄNDERUNG DES CPG GEHALTS**
METHOD FOR MODULATING GENE EXPRESSION BY MODIFYING THE CPG CONTENT
PROCEDE POUR MODULER L'EXPRESSION GENIQUE PAR MODIFICATION DE LA TENEUR EN CPG

(30) Priorität: 03.08.2004 DE 102004037611; 03.08.2004 DE 102004037652
(43) Veröffentlichungstag der Anmeldung: 25.04.2007
(73) Patentinhaber: GENEART AG, 93053 Regensburg (DE)
(72) Erfinder: NOTKA, Frank, 93059 Regensburg (DE); GRAF, Marcus, 93047 Regensburg (DE); LEIKAM, Doris, 93051 Regensburg (DE); WAGNER, Ralf, 93049 Regensburg (DE); RAAB, David, 93176 Beratzhausen (DE)
(74) Vertreter: Grund, Martin
(86) Internationale Anmeldenummer: PCT/EP2005/008423
(87) Internationale Veröffentlichungsnummer: WO 2006/015789

(56) Entgegenhaltungen:
- EP-A- 1 156 112
- WO-A-2004/059556
- DEML L ET AL: "Multiple effects of codon usage optimization on expression and immunogenicity of DNA candidate vaccines encoding the human immunodeficiency virus type 1 Gag protein" JOURNAL OF VIROLOGY, THE AMERICAN SOCIETY FOR MICROBIOLOGY, US, Bd. 75, Nr. 22, November 2001 (2001-11), Seiten 10991-11001, XP002270897 ISSN: 0022-538X
- GRAF ET AL.: "Concerted action of multiple cis-acting sequences is required for rev dependence of late human immunodeficiency virus type 1 gene expression" J. VIROLOGY, Bd. 74, November 2000 (2000-11), Seiten 10822-10826, XP000971716

## Beschreibung

Die vorliegende Erfindung befasst sich mit modifizierten Polynukleotiden, die von natürlich vorkommenden und synthetischen Genen oder anderen kodierenden Sequenzen abgeleitet sind und verglichen mit der ursprünglichen Sequenz eine erhöhte Anzahl von CpG Dinukleotiden in der kodierenden Region aufweisen. Diese Polynukleotide können eingesetzt werden, um die Genexpression zu erhöhen und im speziellen Fall, um die Produktion von Biomolekülen, die Effizienz von DNA-Impfstoffen oder Gentherapiekonstrukten sowie die Qualität von transgenen Tieren oder Pflanzen zu verbessern.

### Hintergrund der Erfindung

Die Bereitstellung von Biomolekülen in Form von Peptiden, Proteinen oder RNA Molekülen ist eine wichtige Komponente im biotechnologischen und pharmazeutischen Sektor. Rekombinant hergestellte oder *in vivo* exprimierte Proteine und RNAs werden sowohl für die Erforschung von grundlegenden Mechanismen und Zusammenhängen als auch zur Produktion von biotechnologischen Reagenzien, zur Herstellung von transgenen Tieren oder Pflanzen oder für medizinische Anwendungen in der Therapie- und Impfstoffentwicklung eingesetzt. Je nach Anwendung sollte die Expressionshöhe entsprechender Moleküle regulierbar sein.

In den meisten Fällen werden Steigerungen über die Standard produktion hinaus angestrebt. Jedes Expressionssystem oder Vektorkonstrukt hat Limitationen, welche die tatsächliche Produktionsleistung bestimmen. Die vorliegende Erfindung betrifft Verfahren und Anwendungen, die geeignet sind, die Expressionshöhe von beliebigen Genen in eukaryontischen Zellen zu modulieren. Insbesondere ist das Verfahren geeignet, beliebige Gene so zu modulieren, dass die erzielbare Genexpression über dem Niveau liegt, das mit bislang bekannten Verfahren zur Steigerung der Expression erzielbar ist.

### Stand der Technik

CpG Dinukleotide nehmen im Genom von Eukaryonten eine besondere Stellung ein. Sie sind nicht wie andere Dinukleotide statistisch verteilt, sondern im direkten Vergleich über weite Strecken des Genoms unterrepräsentiert. Zudem sind CpG Dinukleotide in diesen Bereichen meist methyliert.

Eine Ausnahme hierzu bilden Bereiche, die eine sehr viel höhere Dichte an CpG Dinukleotiden aufweisen und die aufgrund dieser Eigenschaften als CpG Inseln bezeichnet werden. Eine charakteristische Eigenschaft dieser CpG Inseln und eine weitere Abgrenzung zu den CpG Dinukleotiden ist die Tatsache, dass die CpG Dinukleotide in den Inseln in der Regel nicht methyliert vorliegen.

Die Unterrepräsentation von CpG Dinukleotiden wird durch eine chemische Modifikation der entsprechenden Nukleotide erklärt. Im Genom von Vertebraten liegen etwa 60-90 % der Cytosine in CpG Dinukleotiden methyliert vor und diese methylierten Cytosine werden häufig durch Deaminierung zu Thyminen modifiziert (Shen et al., 1994). Dieser Prozess führt dazu, dass die Frequenz an Cytosinen und Guanosinen unter der zu erwarteten statistischen Verteilung bei etwa 40 % liegt und der Anteil an CpG Dinukleotiden sogar nur etwa 20 % der zu erwartenden Häufigkeit aufweist (Bird, 1980; Sved et al., 1990; Takai et al., 2002).

CpG Inseln bilden zu dieser ungewöhnlichen Verteilung von CpG Dinukleotiden eine Ausnahme (Antequera et al., 1993). CpG Inseln sind meistens in der Nähe von Promotoren angesiedelt, können in die transkribierte Region hereinreichen oder sogar innerhalb von Exons liegen.

Sie sind gekennzeichnet durch eine etwa zehnfach höhere CpG Häufigkeit (ca. 60-70 % C+G Gehalt) im Vergleich zu durchschnittlichen Genregionen und vor allem dadurch, dass sie in der Regel nicht methylierte CpGs enthalten (Wise et al., 1999). Etwa 60 % aller humanen Gene, vor allem alle Haushaltsgene und ungefähr die Hälfte der gewebsspezifischen Gene, sind mit CpG Inseln assoziiert (Antequera et al., 1993; Larsen et al., 1992). CpG Inseln sind unter anderem in den Publikationen von Gardiner-Garden M. & Frommer M. (1997) J. Mol. Biol. 196, 261-282 und Takai D. & Jones P. A. (2002) PNAS 99, 3740-3745 beschrieben und definiert worden. Da im Stand der Technik unterschiedliche Definitionen existieren, wird für die Zwecke dieser Erfindung eine CpG Insel wie folgt definiert: Eine CpG Insel umfasst eine Sequenz von mindestens 500 aufeinander folgenden Basenpaaren mit einem CpG Gehalt von mindestens 55 % und einem Quotienten von (tatsächliche CpG / erwartete CpG) von mindestens 0,65, und sie ist mit einem Promoter assoziiert (überlappt ganz oder teilweise mit einem Promotor).

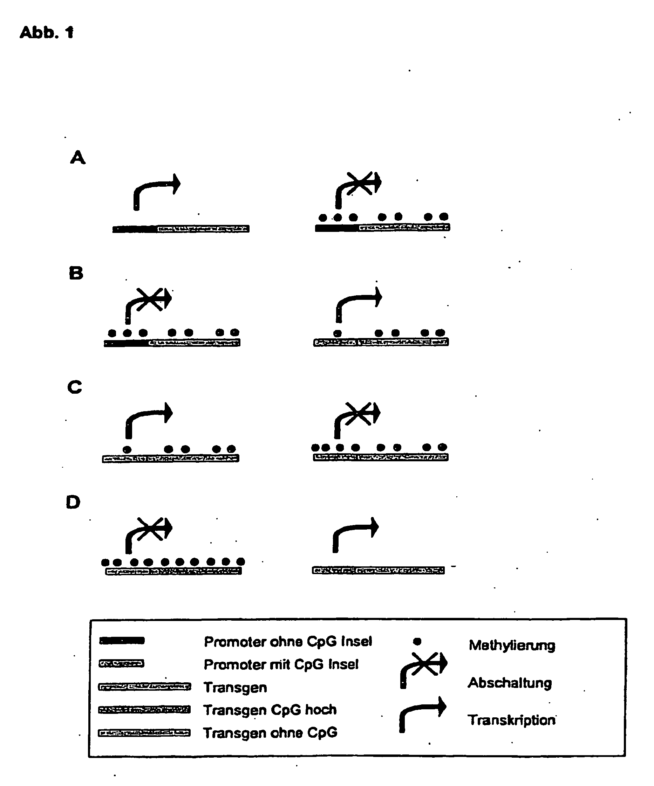

Diese ungleiche Verteilung und Modifikation von CpG Dinukleotiden, i) unterrepräsentiert und methyliert auf der einen Seite und ii) verdichtet und unmethyliert in Insel auf der anderen Seite, hat eine wichtige Kontrollfunktion in der Regulation der Genexpression (schematisch in Abbildung 1 dargestellt).

CpG Dinukleotide sind an der Regulation der Genexpression in frühen Entwicklungsstadien, im Zusammenhang mit der Zelldifferenzierung, dem genetischen Imprinting und anderen Vorgängen beteiligt. In Eukaryonten haben eine Vielzahl von Studien belegt, dass die Methylierung von 5'CpG3' Dinukleotiden (mCpG) einen reprimierenden Effekt auf die Genexpression in Vertebraten und Blütenpflanzen hat (Hsieh, 1994; Kudo, 1998; Jones et al., 1998; Deng et al., 2001; Hisano et al., 2003; Li et al., 2004) (Abb. IA).

Auch in der Tumorforschung gibt es zahlreiche Daten, die belegen, dass i) die Abschaltung der Expression bestimmter Gene, oft Suppressorgene, durch eine Hypermethylierung von CpGs verursacht wird (Li et al., 2004; Kang et al., 2004; Ivanova et al., 2004; Wu et al., 2003) aber auch, dass ii) die unkontrollierte Expression anderer Gene mit einer Hypomethylierung assoziiert ist (Akiyama et al., 2003; Yoshida et al., 2003).

Der Prozess der Genabschaltung durch Methylierung wird durch eine Kaskade von Ereignissen erklärt, die schließlich zu einer Veränderung der Chromatinstruktur führt, die einen transkriptions-schwachen Zustand schafft. Die Methylierung von 5'-CpG-3' Dinukleotiden innerhalb von Genen generiert eine potentielle Bindestelle für Proteinkomplexe (primär aus der Familie der MeCP (*methyl-CpG-binding proteins*) und MBD (*methyl-CpG binding domain protein*) Proteine), welche methylierte DNA Sequenzen binden und gleichzeitig mit Histon-Deacetylasen (MBD-HDAC) und transkriptionellen Repressorproteinen assoziieren (Jones et al., 1998; Nan et al., 1998; Hendrich et al., 1998). Diese Komplexe ziehen in der Regel eine Umstrukturierung des Chromatins nach sich, die zu einer Abschaltung der Transkriptionsaktivität führen (Wade et al., 1999). Die Methylierung in Promotorregionen kann auch direkt zu einer Abschaltung der Genexpression führen, indem die Bindung von essentiellen Transkriptionsfaktoren durch die eingeführten Methylgruppen verhindert wird (Deng et al., 2001).

Die oben beschriebene Deregulation der Expression in Tumorzellen wird im Allgemeinen mit einer Änderung des Methylierungszustandes in den oben beschriebenen CpG Inseln in Zusammenhang gebracht. In normalen Zellen sind aktiv exprimierte Gene meist mit CpG Inseln assoziiert, die nicht oder gering methyliert sind (Abb. IB). Die Methylierung der CpG Dinukleotide in diesen Inseln führt zu einer Abschaltung der Expression dieser Gene (oft Tumor-Supressorgene oder Zellzyklus-Regulatorgene) (Abb. IC) und in der Folge zu einer unkontrollierten Vermehrung dieser Zellen. Im umgekehrten Fall können Gene, die durch eine Methylierung der CpG Dinukleotide in CpG Inseln inaktiv sind, durch eine Demethylierung aktiviert werden.

Die beschriebene Demethylierung in CpG Inseln führt durch eine Änderung der Chromatinstruktur in einen transkriptions-aktiven Zustand, analog der Genabschaltung im Falle einer Methylierung. Zusätzlich zu strukturellen Änderungen kann eine Aktivierung der Expression durch Aktivatorproteine erfolgen. Ein solches zelluläres Aktivatorprotein ist das humane CpG bindende Protein (hCGBP). HCGBP bindet spezifisch an nicht methylierte CpG Dinukleotide im Bereich von Promotoren, wo es als Transaktivator zu einer Erhöhung der Transkription führt (Voo et al., 2000).

Bisher ist die Erkenntnis, dass eine Methylierung der CpG Sequenzen innerhalb eines Gens die Transkription herabreguliert, dazu verwendet worden, die Expression eines Gens, das entweder überexprimiert wird oder dessen Expression unerwünscht ist, durch Methylierung zu verhindern (Choi et al., 2004; Yao et al., 2003) (vgl. Abb. IA).

Eine weitere Anwendung dieser Erkenntnisse ist die gezielte Eliminierung solcher CpG Dinukleotide zur Verbesserung der Genexpression (Chevalier-Mariette et al., 2003). Durch eine Eliminierung wird ebenfalls eine Methylierung und damit verbunden eine Veränderung der Chromatinstruktur hin zu einem transkriptions-inaktiven Zustand verhindert (Abb. ID). In dieser Veröffentlichung wird die Expression eines Transgens mit verschiedenen CpG Dinukleotid-Gehalten in operativer Verknüpfung mit einem Promotor, der sich innerhalb einer CpG-Insel befindet, in Keimbahn-Zellen und den daraus entstandenen Embryonen von transgenen Mäusen untersucht. In diesem speziellen Fall wurde durch die Eliminierung von CpG Dinukleotiden eine transkriptionelle Abschaltung eines Reportergens verhindert (Abb. ID Transgen ohne CpG), wie sie anderenfalls durch eine *de novo* Methylierung von vorhandenen CpG Dinukleotiden während der Embryonalentwicklung zu erwarten ist (Abb. ID, Transgen CpG hoch). Die genauere Untersuchung des Mechanismus in der Veröffentlichung von Chevalier-Mariette zeigte, dass die Verhinderung der Genexpression sowohl mit einer Methylierung der intragenischen CpG Dinukleotide, als auch und vor allem mit einer in der Folge auftretenden Methylierung der Promotor-assoziierten CpG-Insel in Verbindung steht (Abb. ID, Transgen CpG hoch). Für ein Reportergen, das diese intragenischen CpG Dinukleotide nicht aufwies und effizient exprimiert wurde, wurde gezeigt, dass die CpG-Insel nicht methyliert wurde (Abb. ID, Transgen ohne CpG). Die Schlussfolgerung der Autoren war, dass für eine andauernde *in vivo* Expression der CpG Dinukleotid Gehalt in der unmittelbaren Nähe des Promotors und der CpG-Insel vermindert werden muss.

Eine Erhöhung der Genexpression kann ebenfalls durch die Integration kompletter CpG Inseln 5' eines Promotors in entsprechende Vektorkonstrukte erreicht werden (WO 02081677) (vgl. Abb. IB). Bei der Identifizierung des hCGBP wurden ebenfalls CpG Dinukleotide in die entsprechende Promoterregion eines Reportergens integriert und eine Erhöhung der Reporteraktivität festgestellt. In diesen transienten Zellkulturversuchen wurde allerdings das hCGBP ebenfalls überexprimiert und lag daher in nicht physiologischen erhöhten Konzentrationen vor (Voo, et al., 2000).

Es ist bereits bekannt, dass der C/G-Gehalt einen Einfluss auf die mRNA-Stabilität hat. So zeigen beispielsweise Duan und Antezana (2003), dass die Expression von drei verschiedenen Varianten eines humanen Gens in CHO-Zellen Unterschiede in der mRNA-Konzentration zur Folge hat. In der ersten Variante war die humane Gensequenz so verändert worden, dass die Anzahl von C/G-Dinukleodiden maximiert wurde. In einer zweiten Variante war stattdessen die Anzahl der TIA-Dinukleofide maximiert worden. Die Unterschiede im Steady-State-Level, d.h. in der Menge an mRNA, konnten experimentell auf Unterschiede in dem Abbau der mRNA zurückgeführt werden. Aufgrund einer Stabilisierung der Sekundärstruktur bei erhöhtem C/G-Gehalt wurden entsprechende mRNAs weniger stark abgebaut als Wildtyp, und entsprechend wurden T/A-reiche mRNAs in einem viel höheren Maße abgebaut als Wildtyp. Eine Analyse auf Proteinebene wurde nicht durchgeführt, und es ist auch keine Erhöhung der Proteinproduktion durch Erhöhung der CpG Dinukleotide zu erwarten, da eine Stabilisierung der Sekundärstruktur der mRNA die Translation negativ beeinflusst.

Deml et. al. offenbaren eine für die Expression in Säugerzellen kodonoptimierte Sequenz des HIV-I Gag-Gens. Eine spezifische Erhöhung von CpG Dinukleotiden ist nicht offenbart.

EP 1 156 112 beschreibt Kodon-optimierte Nukleinsäuresequenzen, die für gagpol kodieren.

Graf et al. (2000), J. Virology 74:10822-10826 offenbaren die Kodon-optimierte Sequenz "syngag", welche etwa 68 CpG-Dinukleotide enthält.

Ziel der Erfindung war es, ein Verfahren zur gezielten Modulation der Genexpression zu entwickeln, das die Nachteile des Standes der Technik zumindest teilweise beseitigt.

Diese Aufgabe wird gelöst durch ein Verfahren zur gezielten Modulation der Genexpression, umfassend die Schritte:
i. Bereitstellen einer zu exprimierenden Zielnukleinsäuresequenz,
ii. Modifizieren der Zielnukleinsäuresequenz, wobei die Anzahl an in der Zielnukleinsäuresequenz vorhandenen CpG Dinukleotiden unter Verwendung der Degeneration des genetischen Codes zur Erhöhung der Genexpression erhöht wird, wobei die Zielnukleinsäuresequenz so modifiziert ist, dass sie in Bezug auf das verwendete Expressionssystem Kodon-optimiert ist, und dass die Anzahl der CpG-Dinukleotide im Vergleich mit einer Kodon-optimierten, von der Wildtyp-Sequenz abgeleiteten Sequenz unter Verwendung der Degeneration des genetischen Codes erhöht ist,
iii. Klonieren der so modifizierten Zielnukleinsäuresequenz mit modifizierter Anzahl der CpG Dinukleotide in einen geeigneten Expressionsvektor in operativer Verknüpfung mit einer geeigneten Transkriptionskontrollsequenz,
iv. Exprimieren der modifizierten Zielnukleinsäuresequenz in einem geeigneten Säuger-Expressionssystem, wobei das Verfahren kein Verfahren zur therapeutischen Behandlung des menschlichen oder tiersichen Körpers ist.

Vollkommen überraschend hat sich herausgestellt, dass bei Anwendung des Verfahrens der vorliegenden Erfindung genau der entgegengesetzte Effekt erreicht werden kann, als man nach Kenntnis des Standes der Technik erwarten sollte. Das heißt, mit dem Verfahren der vorliegenden Erfindung kann durch die Erhöhung der Anzahl von CpG Dinukleotiden in einer Zielnukleinsäuresequenz die Expression dieser Zielnukleinsäuresequenz erhöht werden. Die Erhöhung der Anzahl von CpG Dinukleotiden im Leserahmen ist dabei erfindungsgemäß nicht mit der Einführung einer CpG Insel gleichzusetzen. Die Erhöhung der CpG Dinukleotide im Leserahmen unterscheidet sich definitionsgemäß von einer CpG Insel durch i) eine mögliche geringere Basenzahl (<500) und ii) das Fehlen einer Überlappung mit dem Promotorbereich.

Bei dem Expressionssystem kann es sich einerseits um eine Zelle oder andererseits um ein zellfreies System oder *in vitro* System handeln. Es wird ein Säuger-Expressionssystem verwendet. Vorzugsweise werden humane Zellen, insbesondere somatische Zellen und keine Keimbahn-Zellen verwendet. Besonders bevorzugt handelt es sich bei dem Expressionssystem um ein System oder um eine Zelle, die methylierungsarm ist, d.h. dass im wesentlichen keine *de novo* Methylierung stattfindet. Andererseits ist es auch möglich, dieses Verfahren - für die Herstellung von transgenen nicht-humanen Organismen, insbesondere von Pflanzen und Tieren, zu verwenden.

Die vorliegende Erfindung betrifft somit insbesondere ein Verfahren zur gezielten Veränderung der Expressionshöhe eines Transkriptes oder/und zur gezielten Veränderung der Protein-Produktion, in SäugerZellen. Das Verfahren ist gekennzeichnet durch Modifikationen des Leserahmens einer zu transkribierenden DNA-Sequenz.

Die Modifikationen betreffen eine Variation des Anteils an CpG Dinukleotiden, die mit einer Veränderung der Expressionshöhe korrelieren.

Die Technologie der artifiziellen Gensynthese ermöglicht es, jede beliebige, aus diesen Möglichkeiten ausgewählte Nukleotidsequenz zu synthetisieren. Durch die Variation von Motiven innerhalb der kodierenden Region eines Gens, welche mit der Expressionshöhe korrelieren, kann mit dieser Technologie die Proteinproduktion gezielt durch die Wahl der entsprechenden Nukleinsäuresequenz moduliert werden. Als solches Motiv mit direktem Einfluss auf die Expressionshöhe wurden im Rahmen der vorliegenden Erfindung CpG Dinukleotide identifiziert.

Überraschenderweise hat sich gezeigt, dass im Gegensatz zur landläufigen Meinung die Einführung von CpG Dinukleotiden auf die erfindungsgemäße Art und Weise statt zu einer Reduktion der Expression zu einer Erhöhung der Genexpression führt. Umgekehrt führt die Eliminierung von CpGs zu einer Reduktion der Genexpression.

Der Ausdruck Genexpression im Sinne der vorliegenden Erfindung umfasst sowohl die Transkription als auch die Translation, insbesondere wird unter diesem Begriff die Proteinproduktion verstanden.

Diese Veränderungen auf Nukleinsäureebene werden im Rahmen dieser Erfindung bevorzugt durch die Herstellung eines artifiziellen Gens durch *de novo* Gensynthese eingeführt, wobei die Aminosäuresequenz, für die das entsprechende Gen kodiert, vorzugsweise unverändert bleibt. *De novo* Gensynthesemethoden sind dem Fachmann auf dem Gebiet bekannt. Die Änderung des CpG-Gehalts erfolgt vorzugsweise durch stumme Mutationen oder durch Mutationen, die die Aktivität des Genprodukts nicht zerstören. Die modifizierten Zielnukleinsäuresequenzen können, wie im Beispiel angegeben, z.B. aus langen Oligonukleotiden mit einer schrittweisen PCR hergestellt werden oder bei gängigen Gensynthese Anbietern (z.B. Geneart GmbH, Qiagen AG) bestellt werden.

Überraschenderweise kann durch die Wahl der Anzahl an CpG Dinukleotiden die Expression des entsprechenden Gens positiv (erhöhte Anzahl an CpG) beeinflusst werden und sogar die Expressionsraten übersteigen, die mit einem kodonoptimierten Gen erreicht werden können. Die Expression kann unerwarteterweise sogar dann gesteigert werden, wenn die Erhöhung der Anzahl der CpG Dinukleotide auf Kosten der RNA- und Kodonoptimierung erfolgt. Vorzugsweise werden bei der Modifizierung der Zielnukleinsäuresequenz keine CpG-Inseln eingefügt, und vorzugsweise ist die modifizierte Zielnukleinsäuresequenz nicht mit CpG-Inseln assoziiert. In Abgrenzung zu den definierten CpG-Inseln, deren Einfluss auf die Expression nach dem alles-oder-nichts Prinzip funktioniert, ergibt sich bei der vorliegenden Erfindung eine Korrelation zwischen Expressionshöhe und Anzahl der CpG Dinukleotide.

Für die Expression von Genen werden diese Modifikationen vorzugsweise so eingeführt, dass die kodierte Aminosäuresequenz nicht verändert wird. Im Idealfall soll nur die Nukleinsäuresequenz eines entsprechenden Gens dessen Expressionshöhe beeinflussen. Da der genetische Code degeneriert ist, besteht die Möglichkeit, für eine bestimmte Aminosäuresequenz eine Vielzahl von korrespondierenden Nukleinsäuresequenzen zu wählen.

In Abgrenzung zu den bisher beschrieben Verfahren soll 1) die für das Transkript kodierende Region modifiziert werden, wodurch dieses Verfahren unabhängig von Vektoren und anderen gentechnischen Vorraussetzungen angewendet werden kann und 2) für eine Steigerung der Expression die Anzahl an CpG Dinukleotiden erhöht werden. Die zusätzlich eingeführten CpGs sind dabei nicht methyliert.

Vorzugsweise wird die Anzahl der CpG Dinukleotide im Vergleich mit der Sequenz der zu exprimierenden Zielnukleinsäure um mindestens 2, vorzugsweise mindestens 3, stärker bevorzugt mindestens 5, stärker bevorzugt mindestens 8, stärker bevorzugt mindestens 10, noch stärker bevorzugt mindestens 15, und bis zu 20 oder noch mehr, insbesondere um 30-50 oder auch bis zu 100 oder darüber, je nach Länge des zu exprimierenden Zielnukleinsäuresequenz, erhöht oder verringert, je nach der gewünschten Expressionshöhe.

Vorzugsweise wird die Anzahl der CpG Dinukleotide im Vergleich mit der Sequenz der zu exprimierenden Zielnukleinsäure um mindestens 10 %, vorzugsweise mindestens 20 %, vorzugsweise mindestens 50 %, vorzugsweise mindestens 100 %, vorzugsweise mindestens 200 % bzw. um das Fünffache oder Zehnfache oder mehr erhöht.

Im Rahmen der vorliegenden Erfindung hat sich überraschenderweise herausgestellt, dass die Erhöhung der Anzahl an CpG Dinukleotiden eine stufenweise Modulation der Genexpression erlaubt. Es wurde überraschenderweise ein Dosiseffekt beobachtet. Das heißt, dass der Grad der Genexpression durch das Hinzufügen von mehr oder weniger CpG Dinukleotiden eingestellt werden kann.

Wie bereits erwähnt, ist es möglich und bevorzugt, sich die Degeneration des genetischen Codes so zunutze zu machen, dass vorzugsweise die maximale Anzahl an CpG Dinukleotiden eingefügt wird, ohne die Aminosäuresequenz der zu exprimierenden Zielnukleinsäuresequenz zu verändern. Die maximale Anzahl an einzufügenden CpG Dinukleotiden ist vorzugsweise begrenzt durch die Variationsmöglichkeiten der degenerierten Kodons einer vorgegebenen Aminosäuresequenz.

Andererseits kann gegebenfalls die Anzahl der CpG Dinukleotide noch darüber hinaus erhöht werden, auch wenn dadurch die korrespondierende Aminosäuresequenz verändert wird. In diesem Fall ist darauf zu achten, dass die Funktion des Peptids bzw. Proteins nicht beeinträchtigt wird.

Die CpG-Dinukleotide können, je nach Art der Degeneration des genetischen Codes, innerhalb eines Kodons oder auch kodonübergreifend hinzugefügt werden.

Zusätzlich zu der Veränderung der Anzahl an CpG Dinukleotiden in der zu exprimierenden Zielnukleinsäure kann letzere je nach dem gewünschten Grad der Genexpression auf Nukleinsäureebene weiter verändert werden. Wenn z. B. eine Erhöhung der Genexpression angestrebt wird, so wird die Anzahl der CpG Dinukleotide vorzugsweise auf eine solche Weise erhöht, dass durch das Einbringen von weiteren CpG Dinukleotiden keine nachteiligen Wirkungen entstehen wie z. B. stärker ausgeprägte Sekundärstrukturen der mRNA, welche sich nachteilig auf die Translation auswirken könnten, weitere Motive, die die Expression negativ beeinflussen, z. B. RNA-Instabilitätsmotive, splice-aktivierende Motive, Endonukleasen-Erkennungsstellen und dergleichen.

Natürlich ist es auch möglich und auch bevorzugt, zusätzlich zu der Erhöhung der Anzahl an CpG Dinukleotiden, darüber hinaus noch eine Nukleinsäure-Optimierung dahingehend vorzunehmen, dass die Genexpression gefördert wird.

Derartige Optimierungen sind demnach das Einfügen oder Entfernen von Motiven, welche die Genexpression beeinflussen können, z.B. Sekundärstruktur-stabilisierende Sequenzabfolgen, Regionen mit erhöhter Selbsthomologie, Regionen mit erhöhter Homologie zum natürlichen Gen, RNA-Instabilitätsmotive, splice-aktivierende Motive, Polyadenylierungs-Motive, adeninreiche Sequenzabschnitte, Endonukleasen-Erkennungsstellen und dergleichen. Noch eine weitere Möglichkeit der Optimierung besteht darin, die Kodonwahl für das gewünschte Expressionssystem jeweils zu optimieren.

Das heißt es kann im Rahmen der vorliegenden Erfindung die Expression noch dadurch erhöht werden, dass zusätzlich zum Einfügen von CpG Dinukleotiden die Kodonwahl optimiert wird. Erfindungsgemäße expressions-optimierte Konstrukte lassen sich beispielsweise erzeugen, indem man die Kodonverteilung so wählt, wie sie in dem verwendeten Expressionssystem auftritt. Bei dem Säuger-Expressionssytem handelt es sich vorzugsweise um ein humanes System. Vorzugsweise wird daher die Kodonoptimierung an die Kondonwahl humaner Gene angepasst. Bevorzugt soll hierbei eine Kodonwahl verwendet werden, wie sie in Säugerzellen am häufigsten oder am zweithäufigsten verwendet wird (Ausubel et al., 1994), um eine generelle Stabilisierung der RNA und eine optimale Kodonwahl zu gewährleisten. Noch mehr bevorzugt wird die Nukleinsäuresequenz unter Verwendung der Gene Optimizer Technologie (DE 102 60 805.9 oder PCT/EP03/14850) für eine optimale Expression modifiziert.

Im Gegensatz zur Kodonoptimierung können aber auch "schlechte", von dem Expressionsystem selten benutzte Kodons verwendet werden, um die Anzahl der CpG Dinukleotiden zu erhöhen.

In dem erfindungsgemäßen Verfahren kann auch eine heterologe Zielnukleinsäuresequenz verwendet werden. Der Ausdruck "heterologe Zielnukleinsäuresequenz" bezieht sich auf die Herkunft der Zielnukleinsäuresequenz und die Herkunft des Expressionssystems. Vorzugsweise sind somit die Zielnukleinsäuresequenz und das Expressionssystem zueinander heterolog, d.h. dass sie entweder aus unterschiedlichen Spezies stammen und/oder dass die Kodonwahl der Wildtyp-Zielnukleinsäuresequenz eine andere ist als die des Expressionssystems. Der Ausdruck heterolog umfasst im Sinne der Erfindung somit auch Unterschiede im Bezug auf die Kodonwahl. Die Kodonwahl bezeichnet den für eine jeweilige Spezies bevorzugten Kodongebrauch im Rahmen der Degeneration des genetischen Codes.

Als Expressionsvektor kann jeder geeignete Expressionsvektor verwendet werden. Solch ein Vektor ist vorzugsweise geeignet für die Expression in Säuger-Zellen. Die modifizierte zu exprimierende Zielnukleinsäure wird so in den Vektor hineinkloniert, dass sie sich in operativer Verknüpfung mit einer geeigneten Transkriptionskontrollsequenz und gegebenenfalls weiteren Regulatorelementen befindet. Eine solche Transkriptionskontrollsequenz kann ein geeigneter Promotor sein, der entweder konstitutiv oder induzierbar sein kann.

Konstitutiv aktive Promotoren sind vorzugsweise ausgewählt aus, aber nicht beschränkt auf CMV (Cytomegalovirus) Promotor und Simian Virus 40 (SV40). Induzierbare Promotoren umfassen, sind aber nicht beschränkt auf Tetracyclin-abhängige Promotoren. Der Fachmann auf dem Gebiet ist ohne Weiteres in der Lage, weitere geeignete Promotoren je nach Anwendung auszuwählen, z.B. auch Promotoren zellulären Ursprungs.

Hierbei ist grundsätzlich jedes induzierbare Promotorsystem geeignet, das im Stand der Technik bekannt ist. Es kann beispielsweise ein natürlicher oder artifizieller induzierbarer Promotor verwendet werden, beispielsweise ein durch Tetracyclin induzierbarer Promotor (Tet on /Tet off-System). Des Weiteren kann aber auch ein induzierbarer viraler Promotor verwendet werden.

Vorzugsweise ist der induzierbare Promotor durch einen transaktiven Faktor induzierbar. Ein viraler induzierbarer Promotor, der durch einen viralen transaktiven Faktor induzierbar ist, kann von einem beliebigen Virus abgeleitet sein. Vorzugsweise werden hierfür Sequenzen von Retroviren, HCV (Hepatitis-C-Virus), HBV (Hepatitis-B-Virus), HSV (Herpes-Simplex-Virus), EBV (Epstein-Barr-Virus), SV 40 (Simian-Virus 40), AAV (Adenoassoziierte Virus), Adenovirus, Papillomviren oder Ebolavirus verwendet. Die hierbei verwendeten transaktiven Faktoren sind demgemäß z.B. ausgewählt aus den folgenden viralen Faktoren, jedoch nicht beschränkt auf diese: NS5A (HCV), HB X (HBV), VP16/ICP4 (EBV), EBNA1/Rta (EBV), ART (HHV8), Large T-Antigen (SV40), Rep78/68 (AAV), E1A (Adenovirus), E2 (Papillomvirus) und VP30 (Ebolavirus).

Als induzierbarer Promotor, der durch einen viralen transaktiven Faktor induzierbar ist, wird vorzugsweise ein retroviraler LTR-Promotor oder eine funktionelle Teilsequenz davon verwendet. Vorzugsweise ist daher der transaktive Faktor ein retrovirales Tat oder Tax Protein. Der LTR-Promotor kann ausgewählt sein aus den LTRs von HIV-1, HIV-2, SIV, HTLV und anderen verwandten Retroviren, die LTR-Promotoren aufweisen. Insbesondere sind lentivirale Promotoren bevorzugt, insbesondere die von HIV.

Vorzugsweise sind die im Rahmen der vorliegenden Erfindung verwendeten Transkriptionskontrollsequenzen, d.h. z.B. Promotoren und/oder Enhancer etc., nicht mit CpG-Inseln assoziiert.

Es ist auch möglich, zusätzlich zur Erhöhung der Anzahl der CpG Dinukleotide in der zu exprimierenden Zielnukleinsäure, die Anzahl der CpG Dinukleotide in den übrigen auf dem Vektor vorhandenen Sequenzen oder Teilen davon zu verringern. Dabei können in diesen übrigen Vektorsequenzen oder Teilen davon die CpG Dinukleotide ganz eliminiert werden. Vorzugsweise geschieht dies wiederum unter Beibehaltung der Aminosäuresequenz unter Ausnutzung der Degeneration des genetischen Codes. Es kann auch nur eine teilweise Eliminierung der CpG Dinukleotide in diesen Sequenzen stattfinden, z.B. um mindestens 5 %, vorzugsweise mindestens 10 %, vorzugsweise mindestens 15 %, vorzugsweise mindestens 25 %, vorzugsweise mindestens 50 %, vorzugsweise mindestens 75 % oder mehr. Vorzugsweise werden alle CpGs entfernt, soweit dies möglich ist.

Somit kann, je nach Anwendung (Silencing oder Expressionssteigerung) die Anzahl an CpG Dinukleotiden unabhängig von der gewählten Kodonoptimierung variiert werden.

In den meisten Fällen ist eine vollständige Eliminierung von CpGs aus dem Leserahmen möglich. Nach oben, d.h. bei der Erhöhung der Anzahl der CpGs, ist die kodierte Aminosäuresequenz limitierend.

Die Zielnukleinsäuresequenz kann für eine RNA, Derivate oder Mimetika davon, ein Peptid oder Polypeptid, ein modifiziertes Peptid oder Polypeptid, ein Protein oder ein modifiziertes Protein kodieren.

Bei der zu exprimierenden Zielnukleinsäure kann es sich bevorzugt um eine Sequenz für ein Gen für ein beliebiges Protein, z.B. ein rekombinantes Protein, ein artifizielles Polypeptid, ein Fusionsprotein und dergleichen, handeln. Bevorzugt sind diagnostische und/oder therapeutische Peptide, Polypeptide und Proteine. Das Peptid/Protein kann z.B. verwendet werden für i) die Herstellung von therapeutischen Produkten, wie z.B. humane Enzyme (z.B. Asparaginase, Adenosin Deaminase, Insulin, tPA, Gerinnungsfaktoren, Vitamin K-Epoxid-Reduktase), Hormone (z.B. Erythropoietin, Follicle-stimulating hormone, Östrogene) und andere Proteine humanen Ursprungs (z.B. bone morphogenetic proteins, Antithrombin), ii) virale, bakterielle oder von Parasiten abgeleitete Proteine, die als Impfstoffe eingesetzt werden können (abgeleitet von HIV, HBV, HCV, Influenza, Borellien, Haemophilus, Meningococcus, Antrax, Botolinus Toxoid, Diphtherie Toxoid, Tetanus Toxoid, Plasmodium, etc.) oder iii) Proteine, die für die Herstellung von diagnostischen Testsystemen verwendet werden können (z.B. Blutgruppenantigene, HLA Proteine).

Als weitere Möglichkeit kann ein Gen gewählt werden, das Botenstoffe (Cytokine/Chemokine) produziert, z.B. G-CSF, GM-CSF, Interleukine, Interferone, PDGF, TNF, RANTES oder MIP1α oder Domänen, Fragmente oder Varianten davon, die geeignet sind, die natürlichen Abwehrmechanismen benachbarter Zellen zu induzieren oder in Kombination mit geeigneten Antigenen eine spezifische Immunantwort zu verstärken.

Eine weitere Anwendungsmöglichkeit ist die Produktion von Proteinen, wie z.B. Enzymen (Polymerasen, Proteasen, etc.) für biotechnologische Anwendungen.

Bei der zu exprimierenden Zielnukleinsäure kann es sich auch um ein Regulatorgen handeln, das nach seiner Expression in einer Zelle als molekulares Schaltermolekül die Expression anderer Gen an- oder abschaltet. Als solches Regulatorgen kann beispielsweise eine Komponente eines Signaltransduktionswegs oder ein Transkriptionsfaktor verwendet werden. Der Begriff "Exprimieren umfasst in diesem Zusammenhang die Transkription der Zielnukleinsäuren und gegebenenfalls die Translation der durch Transkription erhaltenen RNA.

Weiterhin betrifft die vorliegende Erfindung eine modifizierte Nukleinsäure mit einer transkriptionsfähigen Region, die in einem Säuger-Expressionssystem exprimiert werden kann, und die von einer Wildtyp-Sequenz abgeleitet ist, wobei die transkriptionsfähige Region so modifiziert ist, dass sie in Bezug auf das verwendete Säuger-Expressionssystem Kodon-optimiert ist, wobei eine Kodonwahl verwendet wird, wie sie in Säugerzellen am häufigsten oder zweithäufigsten verwendet wird, und dass die Anzahl an CpG Dinukleotiden im Vergleich mit der von der Wildtyp-Sequenz abgeleiteten Sequenz unter Verwendung der Degeneration des genetischen Codes erhöht ist. Die modifizierte Nukleinsäure kann in einem Expressionssystem wie oben beschrieben exprimiert werden, und die transkriptionsfähige Region ist so modifiziert, dass sie in Bezug auf das verwendete Säuger-Expressionssystem Kodon-optimiert ist, und dass die Anzahl der CpG-Dinukleotide im Vergleich mit der Kodon-optimierten, von der Wildtyp-Sequenz abgeleiteten Sequenz unter Verwendung der Degeneration des genetischen Codes erhöht ist, wobei die Anzahl der CpG-Dinukleotide im Vergleich mit der Wildtyp-Sequenz um mindestens das Fünffache, vorzugsweise um das Zehnfache oder mehr erhöht ist.

Eine Wildtyp-Sequenz im Sinne dieser Erfindung ist eine Nukleinsäuresequenz, die natürlich vorkommt.

Wie oben bereits erwähnt, ist es jedoch auch möglich, dass die Zielnukleinsäuresequenz für eine zusammengesetzte Gensequenz kodiert, welche aus unterschiedlichen Wildtyp-Sequenzen zusammengesetzt sein kann. In einem solchen Fall bezieht sich Wildtyp-Sequenz auf die Sequenz, welche noch nicht im Sinne der vorliegenden Erfindung modifiziert wurde (Erhöhung oder Verringerung der Anzahl der CpG-Dinukleotide).

Vorzugsweise ist die Anzahl auf die maximale Anzahl erhöht, die im Rahmen der Degeneration des genetischen Codes möglich ist.

Ein weiterer Gegenstand der Erfindung ist die Bereitstellung eines Expressionsvektors, der eine oben erwähnte erfindungsgemäße modifzierte Nukleinsäure in operativer Verknüpfung mit geeigneten Transkriptionskontrollsequenzen umfasst. Der Vektor wird vorzugsweise für eine Erhöhung der Expression in Säuger-Zellen einer beliebigen DNA Sequenz eingesetzt. Der Vektor ist vorzugsweise von bekannten Vektoren abgeleitet. In den Sequenzbereichen des Vektors, die von der erfindungsgemäßen modifizierten Nukleinsäuresequenz verschieden sind, ist vorzugsweise die Anzahl der CpG Dinukleotide verringert. Vorzugsweise ist die Anzahl an CpG Dunkleotiden in diesen übrigen Vektorsequenzen oder Teilen davon um mindestens 5 %, vorzugsweise mindestens 10 %, vorzugsweise mindestens 15 %, vorzugsweise mindestens 25 %, vorzugsweise mindestens 50 %, vorzugsweise mindestens 75 % oder mehr verringert.

Die Reduktion von CpGs wird vorzugsweise durch artifizielle Gensynthese der einzelnen Vektormodule (Antibiotikumsresistenzgen, Selektionsmarker, Multiple cloning site etc.) wie oben beschrieben, erreicht. Die einzelnen Module werden mit entsprechenden DNA Fragmenten essentieller, nicht veränderbarer Module (Replikationsursprung, Polyadenylierungsstelle, viraler Promotor etc.) unter Verwendung singulärer Restriktionsstellen zu einem funktionellen Vektor zusammengesetzt. Bei dem Vektor kann es sich um einen viralen (z.B. abgeleitet von Adenoviren, Retroviren, Herpesviren, Alphaviren etc.) oder bakteriellen Ursprungs oder nackte DNA (Expressionsplasmide) handeln.

Der modulare Aufbau des Vektors erlaubt darüber hinaus eine schnell und einfach durchzuführende Veränderung bezüglich der einzelnen Module. Die Anzahl der Module kann variiert und entsprechend der Anwendung angepasst werden.

Für eine stabile Integration in Zellen können Elemente, wie eukaryontische Selektionsmarker (z.B. Resistenzgene gegen Hygromycin, Zeocin etc.; Selektionsreporter, wie GFP, LNGFR etc; oder Rekombinationssequenzen für eine gerichtete Rekombination) eingesetzt werden, wobei die entsprechenden Gensequenzen, soweit möglich, ebenfalls im Gehalt von CpGs reduziert werden können. Für Anwendungen in der Gentherapie können Sequenzen eingeführt werden, die immunstimulierenden Motiven entgegen wirken (z.B. immunreprimierende CpG Motive). Entsprechend können für Anwendungen bei Immunisierungen, wie z.B. bei Impfungen oder zur Herstellung von Antikörpern, Sequenzen integriert werden, welche immunstimulierende Faktoren (z.B. Immunstimulierende CpG Motive) enthalten.

Ein für diese Erfindung bevorzugter Vektor ist der in SEQ ID NO. 27 dargestellte Vektor.

Ein weiterer Gegenstand der vorliegenden Erfindung sind eukaryontische Zellen, stärker bevorzugt Säugetierzellen, am meisten bevorzugt humane Zellen; die eine Zielnukleinsäure oder einen Vektor (vorzugsweise in Form eines DNA-Konstrukts) wie oben beschrieben enthalten, wobei die Nukleinsäure oder der Vektor in einer transkriptionsfähigen Form vorliegen. Die Zellen sind vorzugsweise somatische Zellen bzw. bevorzugt solche Zellen, die im wesentlichen keine de novo Methylierung durchführen.

Das DNA-Konstrukt kann beispielsweise episomal vorliegen oder stabil ins Chromosom integriert sein. Dabei können in der Zelle ein oder mehrere Kopien vorliegen. Für das Einbringen besagter DNA-Konstrukte können Genfähren viralen (z.B. Adenoviren, Retroviren, Herpesviren, Alphaviren etc.) oder bakteriellen Ursprungs oder nackte DNA (Expressionsplasmide) verwendet werden.

Offenbart ist ferner ein Säuger-Expressionssystem, umfassend
a) eine modifizierte Nukleinsäuresequenz mit einer transkriptionsfähigen Region, die von einer Wildtyp-Sequenz abgeleitet ist, wobei die modifizierte Nukleinsäuresequenz so modifiziert ist, dass sie in Bezug auf das verwendete Säuger-Expressionssystem Kodon-optimiert ist und eine im Vergleich mit der Kodon-optimierten Sequenz erhöhte oder verringerte Anzahl an CpG Dinukleotiden aufweist, in operativer Verknüpfung mit einer Transkriptionskontrollsequenz und
b) eine Expressionsumgebung, ausgewählt aus einer Säugetierzelle und einer zellfreien Expressionsumgebung, worin (a) exprimiert werden kann, wobei das Säuger-Expressionssystem bei Expression einer modifizierten Nukleinsäuresequenz mit erhöhter Anzahl an CpG Dinukleotiden eine erhöhte Expression zeigt und bei Expression einer modifizierten Nukleinsäuresequenz mit verringerter Anzahl an CpG Dinukleotiden eine verringerte Expression zeigt.

Die vorliegende Erfindung kann somit verwendet werden, um die Expression einer Zielnukleinsäuresequenz zu erhöhen. Wird die Expression gesteigert, so sollte vorzugsweise eine Erhöhung der Expression um mindestens 5 %, bevorzugt um mindestens 10 %, bevorzugt mindestens 20 %, stärker bevorzugt mindestens 30 %, noch stärker bevorzugt mindestens 50 %, noch stärker bevorzugt mindestens 100-400 % oder mehr erfolgen. Je nach Länge der zu exprimierenden Zielnukleinsäuresequenz und der Anzahl der CpG Dinukleotide, die eingefügt werden können, kann auch eine Steigerung der Expression um das Zweifache, Dreifache, Fünffache oder sogar Zehnfache bis Zwanzigfache oder sogar bis Einhundert- bis Zweihundertfache erreicht werden.

Wie oben bereits ausführlich dargelegt, hängt die Höhe der Transkription von der Anzahl der CpG Dinukleotide im Gen ab. Das heißt, dass bei längeren Genen oder bei Genen mit mehr Möglichkeiten CpG Dinukleotide einzufügen, eine höhere Steigerung der Expression zu erreichen ist.

Ein weiterer Gegenstand der vorliegenden Erfindung sind Arzneimittel und Diagnostika auf der Grundlage der erfindungsgemäßen modifizierten Nukleinsäuren und/oder Vektoren. Die modifzierten Nukleinsäuren und Vektoren können bei diagnostischen, therapeutischen und/oder gentherapeutischen Anwendungen eingesetzt werden, insbesondere auch zur Herstellung von Impfstoffen.

Insbesondere können das erfindungsgemäße Verfahren und die erfindungsgemäßen Expressionssysteme, Nukleinsäuresequenzen, Vektoren und Zellen zur Herstellung von DNA-Impfstoffen verwendet werden. Alternativ zu herkömmlichen Tot- und Lebendimpfstoffen gewinnt die Entwicklung von Impfstoffen, die auf "nackter" Plasmid-DNA basieren, immer mehr an Bedeutung. Der Vorteil der DNA-Impfstoffe liegt in einer Aufnahme der DNA in Zellen, verbunden mit der authentischen Produktion (inklusive Modifikation) der Antigene und einer effizienten Aktivierung von zellulärer und humuraler Immunantwort. Dabei korreliert die Höhe der induzierten Immunantwort mit der produzierten Antigenmenge und damit mit der Expressionsleistung der DNA-Konstrukte. Wenn die Expression eines beliebigen Antigens durch die Anreicherung von CpG Dinukleotiden in der kodierenden Sequenz erhöht werden kann, wird in der Folge die Aktivierung des Immunsystems und damit die Schutzwirkung verbessert.

### Beschreibung der Abbildungen

### Abb. 1:

### Regulation der Genexpression durch Methylierung (Stand der Technik).

A: Methylierung von CpG Dinukleotiden führt zur Abschaltung der Genexpression.
B: CpG Inseln schützen vor einer Methylierung und der damit verbundenen Abschaltung.
C: Sekundäre Hypomethylierung der CpG Inseln führt zu einer Genabschaltung.
D: Sekundäre Hypomethylierung kann verhindert werden durch die Reduktion von CpG Dinukleotiden im Leserahmen.

### Abb. 2:

### GFP Expressionsanalyse in stabil transfizierten Zellen.

A und B: Langzeit Durchfluss-Zytometrie Analyse stabil transfizierter Flp-In 293T und CHO Zellen. Die Y-Achse gibt die GFP-bedingte Fluoreszenzintensität (MFI "mean fluorescence intensity") und die x-Achse die Messzeitpunkte in Wochen nach Transfektion.
A: FACS Analyse von huGFP und ΔCpG-GFP rekombinanten 293T Zellen.
B: FACS Analyse von huGFP und ΔCpG-GFP rekombinanten CHO Zellen.
C: Fluoreszenzmikroskopische Aufnahme stabiler Zelllinien.

### Abb. 3:

GFP Proteinnachweis in stabil transfizierten Zellen. Expressionanalyse der GFP Leserahmen. Rekombinante Flp-In CHO Zellen, die das huGFP oder das ΔCpG-GFP Gen stabil im Zellgenom integriert haben, wurden lysiert und die Expression der Gene wurde durch konventionelle Immunoblot Analysen nachgewiesen. Auftrag der huGFP, ΔCpG-GFP und Mock-Proben sind angegeben. Von beiden polyklonalen Zellkulturen (poly.) wurden monoklonale Zelllinien etabliert (mono. 14 und 7 für ΔCpG-GFP und mono. 10 und 9 für huGFP). Mock-Zellen entspricht einer unveränderten Ausgangszellpopulation.

### Abb. 4:

Quantitative Bestimmung spezifischer Transkripte stabiler Zellen. Realtime PCR Analyse spezifischer Hygromycin-Resistenzgen und gfp RNAs aus cytoplasmatischen RNA Präparationen. Die Realtime PCR Auswertung der LC Analysen sind für CHO Zellen (Hygromycinresistenz A und gfp B) sowie für 293T Zellen (Hygromycinresistenz C und gfp D) dargestellt. Abgebildet sind die Anzahl der PCR Zyklen (X-Achse) und die Fluoreszenzintensität (Y-Achse). Die spezifischen Kinetiken sind für huGFP- und ΔCpG-GFP-Produkte sowie für die primer dimer angezeigt.

### Abb. 5:

MIP1alpha Expressionsanalyse nach transienter Transfektion. Repräsentative ELISA Analyse der Zelllysate und Überstände transfizierter H1299 Zellen. H1299 Zellen wurden mit jeweils 15 µg Wildtyp-und optimierten murinen MIP1alpha Konstrukten transfiziert. Die jeweilige Proteinkonzentration wurde durch konventionelle ELISA Tests im Zellkulturüberstand und im Zelllysat anhand entsprechender Standardkurven quantifiziert. Die Balken repräsentieren den Mittelwert der Gesamtproteinkonzentration für jeweils 2 unabhängige Ansätze, die Fehlerbalken entsprechen der Standardabweichung. Auf der X-Achse ist die Anzahl der CpG Dinukleotide im offenen Leserahmen und auf der Y-Achse die Gesamtproteinkonzentration in pg/ml angegeben. Wt entspricht dem Expressionskonstrukt des jeweiligen Wildtyp-Gens.

### Abb. 6:

MIP1alpha und GM-CSF Expressionsanalyse nach transienter Transfektion. Repräsentative ELISA Analyse der Überstände transfizierter H1299 Zellen. H1299 Zellen wurden mit jeweils 15 µg Wildtyp- und optimierten humanen MIP1alpha (A) und GM-CSF (B) Konstrukten transfiziert. Die jeweilige Proteinkonzentration im Überstand der Zellkultur 48h nach Transfektion wurde durch konventionelle ELISA Tests entsprechender Standardkurven quantifiziert. Die Balken repräsentieren den Mittelwert für jeweils 2 unabhängige Ansätze, die Fehlerbalken entsprechen der Standardabweichung. Auf der X-Achse ist die Anzahl der CpG Dinukleotide im offenen Leserahmen und auf der Y-Achse die Proteinkonzentration im Überstand in pg/ml angegeben. Wt entspricht dem Expressionskonstrukt des jeweiligen Wildtyp-Gens.

### Abb. 7:

### Schematische Darstellung der verwendeten Expressionsplasmide.

A: Plasmidkarte des P-smallsyn Plasmides.
B: Plasmidkarte des Pc-ref. Module und Herkunft der Sequenzen (Wildtyp "Wt" in schwarz und synthetisch in grau) sind angegeben.

### Abb. 8:

HIV-1 p24 Nachweis nach transienter Transfektion. Expressionanalyse der P-smallsyn und Pc-ref Vektoren. H1299 Zellen wurden mit den angegebenen Konstrukten transfiziert und die Proteinproduktion wurde durch konventionelle Immunoblot Analysen nachgewiesen. Analyse der Zelllysate von HIV-1 p24 transfizierten H1299 Zellen. Molekulargewichte (precision plus protein standard, Bio-Rad) sowie Auftrag der R/p24, s/p24 und Mock-transfizierten Proben sind angegeben. Mock-Transfektion entspricht einer Transfektion mit ursprünglichem pcDNA3.1 Plasmid.

### Abb. 9:

HIV-1 p24-Expressionsanalyse verschiedener Expressions-konstrukte. H1299 Zellen wurden mit jeweils 15 µg R/p24, R/p24ΔCpG, s/p24 und s/p24ΔCpG Konstrukten sowie mit pcDNA3.1 (Mock Kontrolle) in unabhängigen Zweifachansätzen transfiziert. Die jeweilige p24-Proteinkonzentration im Zelllysat wurde durch konventionelle Immunoblot Analysen (A) und durch ELISA Tests (B) anhand entsprechender Standardkurven quantifiziert. Die Balken repräsentieren den Mittelwert der p24 Konzentration (in µg/ml) im Zelllysat für jeweils 2 unabhängige Ansätze.

### Beispiele

Beispiele, die nicht unter den Schutzbereich der Ansprüche fallen, dienen nur illustrativen Zwecken.

### Beispiel 1

### Herstellung von GFP-Reportergenen mit unterschiedlichem CpG-Gehalt

Zwei Varianten an Grünfluoreszenz-Protein (GFP) Genen, die sich in der Anzahl an CpG Dinukleotiden unterscheiden, wurden hergestellt. Das huGFP Gen wies 60 CpGs auf, das ACpG-GFP Gen hatte keine CpGs. Das CpG depletierte Gen ΔCpG-GFP wurde künstlich aufgebaut. Beim Design des ΔCpG-GFP wurde darauf geachtet, keine seltenen Kodons oder negativ wirksamen cis-aktiven Elemente wie Spleißstellen oder poly(A) Signalstellen einzufügen. Der codon adaptation index (CAI), ein Maß für die Güte der Kodonwahl, wurde durch die Depletierung der CpGs nur geringfügig verändert (CAI(huGFP) = 0,95; CAI(ΔCpG-GFP) = 0,94). Die kodierende Aminosäuresequenz des GFP wurde dabei nicht verändert. Zur Subklonierung wurden weitere Schnittstellen eingefügt. Die Nukleotid- und Aminosäuresequenz sind in SEQ ID NO. 1/2 angegeben.

Die Sequenz wurde als vollsynthetisches Gen hergestellt (Geneart GmbH), unter Verwendung der Schnittstellen *Hind*III und *Bam* HI in den Expressionsvektor pcDNA/5FRT (Invitrogen) kloniert und unter die transkriptionelle Kontrolle des Cytomegalo-Virus (CMV) frühen Promotor/Enhancer gestellt ("pc-ΔCpG-GFP").

Zur Herstellung eines analogen, jedoch in seiner CpG Verteilung unveränderten GFP Expressionsplasmides, wurde die kodierende Region des humanisierten GFP Genes (huGFP) mittels Polymerase Kettenreaktion (PCR) unter Verwendung der Oligonukleotide huGFP-1 und huGFP-2 aus einem kommerziell erhältlichen Vektor amplifiziert und ebenfalls unter Verwendung der Schnittstellen *Hind*III und *Bam* HI in den Expressionsvektor pcDNA/5FRT einkloniert ("pc-huGFP", SEQ ID NO. 3/4).

### Herstellung von stabilen Zelllinien mit den GFP Genvarianten

Für eine schnelle Etablierung und Selektion von stabilen, rekombinaten Zellen wurde das Flp-In System von Invitrogen verwendet. Ein weiterer, wesentlicher Vorteil dieses Systems besteht in einer gerichteten Integration einer Kopie des Transgens in einen definierten Lokus der Zielzelle. Diese Technologie schafft damit die beste Vorraussetzung für den quantitativen Vergleich der Expression eines beliebigen Transgens, da physiologische und genetische Faktoren der Zielzelle weitestgehend identisch sind. Um eine zusätzliche Absicherung zu erreichen, wurden zwei unterschiedliche Säugetierzellen für diese vergleichenden Analysen ausgewählt. Die Zelllinien Flp-In CHO und Flp-In 293T wurden von Invitrogen bezogen und bei 37°C und 5 % CO₂ kultiviert. Die Zelllinien wurden in Dulbecco's Modifiziertem Eagle Medium high glucose (DMEM) (293T) und HAMs F12 (CHO) mit L-Glutamin, 10 % inaktiviertem fötalem Rinderserum, Penicillin (100 U/ml) und Streptomycin (100 µg/ml) kultiviert. Die Zellen wurden nach Erreichen der Konfluenz im Verhältnis 1:10 subkultiviert.

Die Etablierung stabil transfizierter Zellen wurden gemäß der Angaben des Herstellers durchgeführt. 2,5 x 10⁵ Zellen wurden in 6-well Kulturschalen ausgesät und 24 h später durch Calciumphosphat Kopräzipitation (Graham and Eb, 1973) mit 1,5 µg Transferplasmid und 13,5 µg pOG44 transfiziert. Zellen wurden bis zu einem Verhältnis von >90 % GFP positive Zellen mit 100 µg/ml Hygromycin für 293T und 500 µg/ml für CHO Zellen selektioniert. Die Anzahl an GFP positiven Zellen wurde für alle Zelllinien mittels konventioneller Durchfluss-Zytometrie Analyse bestimmt.

### Bestimmung der GFP Expression

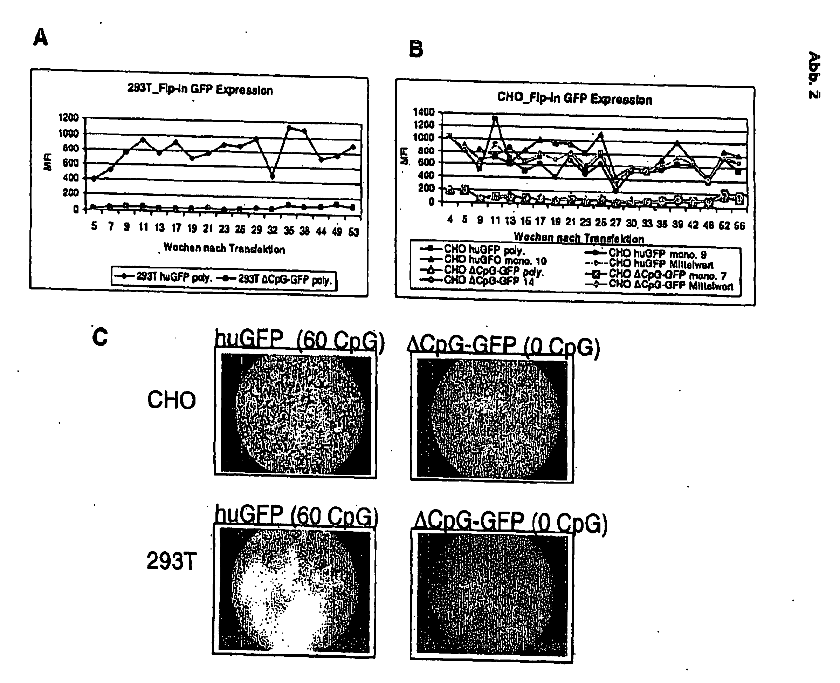

Die Expression der Reporterkonstrukte wurde über einen Zeitraum von 16 Monaten durch regelmäßige Messung der GFP-vermittelten grünen Autofluoreszenz in einem Durchfluss-Zytometer (Becton-Dickinson) bestimmt. Die Angaben der mittleren Fluoreszenzintensitäten sind in Abb. 2A (293T Zellen) und 2B (CHO Zellen) zusammengefasst. Die huGFP-Expression konnte über den gesamten Messzeitraum in beiden Zelllinien relativ konstant mit einer mittleren Fluoreszenz-Intensität von 800 (293T) bzw. 700 (CHO) nachgewiesen werden. Das in der Anzahl an CpGs reduzierte ΔCpG-GFP Reporterkonstrukt konnte ebenfalls über den gesamten Messzeitraum in konstanter Fluoreszenzintensität nachgewiesen werden. Die mittlere Fluoreszenzintensität war jedoch verglichen mit dem huGFP um den Faktor 10-20 (293T) bzw. 6-9 (CHO) reduziert. Die Reduktion der GFP-vermittelten Fluoreszenz war auch im Fluoreszenzmikroskop nachweisbar (Abb. 2C).

Da für einen Abfall der GFP-vermittelten Fluoreszenz unterschiedliche Ursachen in Betracht gezogen werden können (Instabilität des Proteins, reduzierter Kemexport der RNA, geringere Transkriptionsrate etc.) wurden zusätzlich Western Blot Analysen und quantitative Realtime PCR durchgeführt.

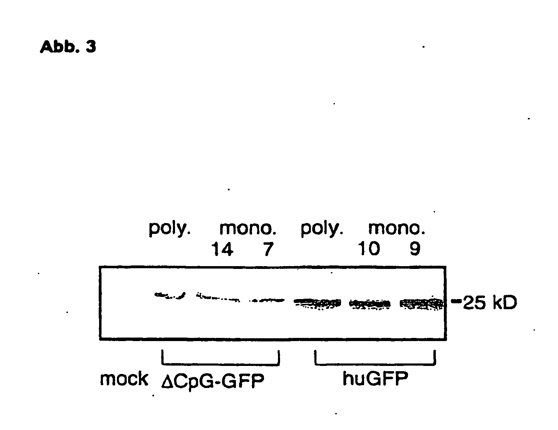

Für den Proteinnachweis durch Immunoblot wurden die stabilen transfizierten CHO Zellen zweimal mit eiskaltem PBS (10 mM Na₂HPO₄, 1,8 mM KH₂PO₄, 137 mM NaCl, 2,7 mM KCI) gewaschen, in eiskaltem PBS abgekratzt, 10 Min. bei 300 x g abzentrifugiert und in Lyse-Puffer (50 mM Tris-HCl, pH 8,0, 0,5 % Triton X-100 (w/v)) 30 min auf Eis lysiert. Unlösliche Bestandteile des Zellysates wurden 30 min bei 10000 x g und 4 °C abzentrifugiert. Die Gesamtproteinmenge des Überstandes wurde mit dem Bio-Rad Protein Assay (Bio-Rad, München) nach Herstellerangaben bestimmt. Die Proben wurden mit gleichem Volumen an 2-fach Probenpuffer (Laemmli, 1970) versetzt und 5 min auf 95 °C erhitzt. 40 µg Gesamtprotein aus Zelllysaten wurden über ein 12,5 %iges SDS/Polyacrylamidgel aufgetrennt (Laemmli, 1970) auf eine Nitrocellulose-Membran elektrotransferiert und mit einem monoklonalen GFP-spezifischen Antikörper (BD-Bioscience) und einem sekundären, HRP (Meerrettich-Peroxidase: horse-radish-preoxidase) gekoppelten Antikörper detektiert und mittels chromogener Färbung nachgewiesen. Der Proteinnachweis per Western Blot bestätigte die Daten aus der FACS Messung. Für beide Genvarianten wurde in stabil transfizierten CHO Zellen das Volllänge-GFP Protein nachgewiesen, Unterschiede in der Prozessierung oder proteolytischen Degradation waren nicht nachzuweisen (Abb. 3).

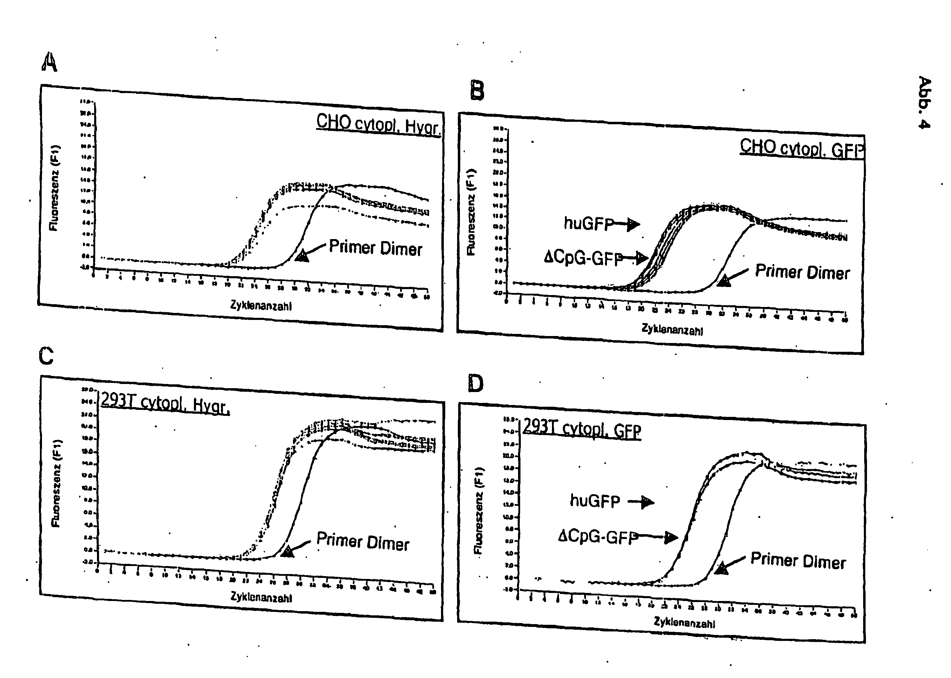

Für eine Aufklärung der Transkriptionsaktivität wurde eine quantitative Realtime PCR (Light Cycler, Roche) für die stabil transfizierten CHO Zellen durchgeführt. Cytoplasmatische RNA wurde aus den Zellen präpariert (RNeasy, Quiagen) und mit DNase behandelt (500 U RNase-freie DNase / 20 µg RNA). 1 µg der DNase-behandelten RNA wurde als Matrize für eine Reverse Transcription (Random Primed, p(dN)₆, 1st strand C-DNA Synthesis kit for RT-PCR, Roche) mit anschließender PCR (RT-oligo1 und RT-oligo2) eingesetzt. Das resultierende PCR Produkt wurde verdünnt und für eine light cycler (LC) Analyse (SYBR, Roche) eingesetzt. Als interne Kontrolle wurde die RNA Menge des ebenfalls in das Zellgenom integrierten Hygromycinresistenzgens bestimmt. Die Ergebnisse sind in Abb. 4 zusammengefasst. Die RNA Mengen der Hygromycinresistenz zeigten keinen Unterschied in allen gemessenen Konstrukten (Abb. 4A für CHO Zellen und 4C für 293T Zellen). Die Ergebnisse der GFP RNA korrelierten dagegen sehr gut mit den Ergebnissen der Proteinexpression (GFP Fluoreszenzintensität). Für das CpG-deletierte Konstrukt war nach Quantifizierung der Light Cycler Daten im Vergleich zu dem Ausgangskonstrukt eine etwa siebenfach geringere cytoplasmatische RNA-Menge in CHO Zellen (Abb. 4B) und eine etwa dreißigfach geringere RNA-Menge in 293T Zellen (Abb. 4D) nachzuweisen.

### Beispiel 2

### Herstellung von murinen Mip1alpha Genen mit unterschiedlichem CpG-Gehalt

In diesem Beispiel wurde die Nukleinsäuresequenz des murinen MIP1alpha Gens so verändert, dass eine Reihe von Konstrukten mit unterschiedlicher Anzahl von CpG Dinukleotiden entstand, ohne jedoch die kodierende Aminsäuresequenz zu verändern. Dazu wurde die Aminosäuresequenz des murinen MIP1alpha Genproduktes in synthetische MIP1alpha-kodierende Leserahmen, unter Verwendung der Kodonwahl humaner Zellen, rückübersetzt. In einer ersten Serie von Konstrukten wurden die zufällig entstanden CpG Dinukleotide wieder schrittweise aus der Sequenz entfernt, ohne jedoch seltene Kodons, die eine Expression erwartungsgemäß verschlechtern würden, einzufügen. Darüberhinaus wurde ein CpG-Dinukleotid optimiertes MIP1alpha Genkonstrukt hergestellt, welches doppelt soviele CpG Dinukleotide wie das kodonoptimierte Konstrukt aufwies. In diesem Falle wurde bewusst eine Verschlechterung der Kodonwahl in Kauf genommen, um möglichst viele CpG Dinukleotide einzufügen. Nach Stand der Technik wäre zu erwarten, dass dieses Genkonstrukt auf Grund seiner schlechteren Kodonwahl eine geringere Expression als das kodonoptimierte Genkonstrukt aufweisen würde.

Diese Genvarianten wurde unter Verwendung von langen Oligonukleotiden und einer schrittweisen PCR als vollsynthetische Leserahmen aufgebaut und in einen Expressionsvektor kloniert. Die hergestellte MIP1 alpha Vektorvarianten erwiesen sich in der Expressionshöhe des (murinen MIP1alpha als vollständig unterschiedlich. Für den Fachmann nicht vorhersehbar zeigte sich, dass die Varianten mit den geringsten CpGs am schlechtesten exprimiert wurden und mit der Erhöhung der CpGs eine Steigerung der MIP1alpha Expression in Säugerzellen einherging. Insbesondere war für den Fachmann nicht vorhersehbar, dass das Konstrukt mit der maximal möglichen Anzahl an CpG Dinukleotiden, die jedoch auf Kosten einer Verschlechterung der Kodonwahl eingeführt wurden, eine signifikant stärkere Expression aufwies, als das kodonoptimierte Gen.

Varianten des murinen Mip1alpha Gens, die sich in der Anzahl an CpG Dinukleotiden unterscheiden, wurden wie unter Beispiel 1 beschrieben künstlich aufgebaut und unter Verwendung der Schnittstellen *Hind*III und *Not*l in den Expressionsvektor pcDNA3.1 subkloniert. Die künstlich hergestellten Gene wurden in ihrer Kodonwahl jeweils an das Säugersystem angepasst. Beim Entfernen der CpG Dinukleotide wurden keine seltene Säugerkodons verwendet, beim Einfügen von CpG Dinukleotide über die Anzahl von Dinukleotiden die mit einer normalen Kodonadaptierung erreicht werden, wurden bewusst auch seltene Kodons verwendet.

Die kodonoptimierten, jedoch mit unterschiedlicher Anzahl von CpG Dinukleotiden versehenen Konstrukte wiesen durchweg einen CAI-Wert von über 0,9 auf und unterschieden sich nur unwesentlich. Die CAI Werte des Wildtyp-Gens, als auch des CpG Dinukleotid optimierten Gens (42 CpGs) wiesen dagegen sehr niedrige CAI Werte (unter 0,8) auf. Nach Stand der Technik wäre also eine vergleichbare Expression der kodonoptimierten Gene zu erwarten, jedoch eine deutlich geringere Expression des Wildtypgens und des CpG Dinukleotid optimierten Gens. Die Bezeichnung der Konstrukte, die Anzahl der CpGs sowie die CAI Werte sind in Tabelle 1 wiedergegeben. Die Nukleotid- und Aminosäuresequenzen sind in SEQ ID NO. 5/6 bis SEQ ID NO. 13/14 angegeben. Das analoge, der Wildtyp-Sequenz entsprechende Expressionskonstrukt (Wildtyp-Referenzkonstrukt), war in seiner CpG Verteilung unverändert. Die kodierende Region wurde mittels Polymerase Kettenreaktion (PCR) unter Verwendung der Oligonukleotide mamip-1 und mamip-2 aus einem cDNA Klon (von RZPD bezogen) amplifiziert und ebenfalls unter Verwendung der Schnittstellen *Hind*III und *Not*l in den Expressionsvektor pcDNA3.1 einkloniert ("pc-mamipwt", SEQ ID NO. 15, GenBank Accession Nummer AA071899).

### Überprüfung der Mip1alpha Expression

Zur Quantifizierung der Chemokin-Expression wurden humane H1299 Zellen mit den jeweiligen Expressionskonstrukten transfiziert und die Proteinmenge in den Zellen und im Zellkulturüberstand mittels kommerzieller ELISA Testkits gemessen.

1,5 x 10⁵ humane Lungenkarzinomzellen (H1299) wurden in 6-well Zellkulturschalen ausgesät und 24 h später durch Calciumphosphat Präzipitation mit 15 µg des entsprechenden Expressionsplasmids transfiziert. Zellen und der Zellkulturüberstand wurden 48 h nach der Transfektion geerntet. Die transfizierten Zellen wurden wie im Beispiel 1 beschrieben lysiert und die Gesamtproteinmenge des Zelllysates wurde mit dem Bio-Rad Protein Assay bestimmt. Aus dem Zellkulturüberstand wurden durch Zentrifugation bei 10000 x g für 15 min bei 4 °C unlösliche Zellbestandteile entfernt.

Aus 1-5 µg Gesamtprotein aus Zellysaten sowie aus verdünnten Zellkulturüberständen wurde jeweils in einem kommerziell erhältlichen ELISA Assay (R & D Systems), die Expression des Mip1alpha nach den Angaben des Herstellers überprüft. Vergleichbar mit den Daten der GFP- und p24-Expressionskonstrukten, korrelierte die Gesamtmenge an detektierbaren Mip1alpha mit der Anzahl an CpGs im Leserahmen. Die Daten sind in Tabelle 1 zusammengefasst. Die Anzahl der Konstrukte erlaubte erstmals eine detaillierte Auswertung des Zusammenhangs der Expressionshöhe mit der Anzahl an CpGs innerhalb der kodierenden Region.

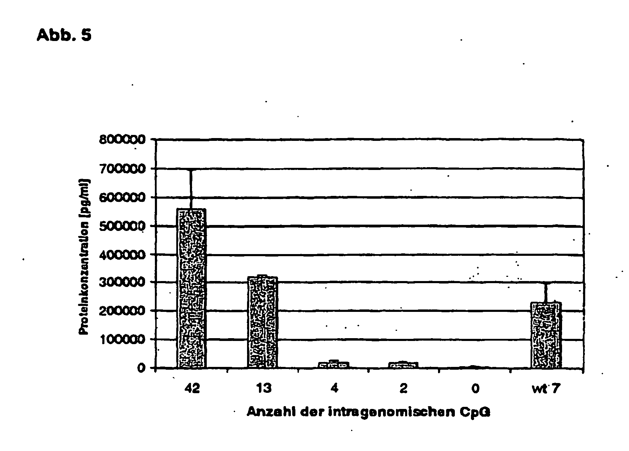

Ein repräsentatives Ergebnis einer Auswertung mittels Cytokin ELISA ist in Abbildung 5 wiedergegeben. Die Balken entsprechen dem Mittelwert zweier unabhängiger Transfektionsansätze, die Fehlerbalken repräsentieren die jeweilige Standardabweichung.

In der Tabelle 1 sind die relativen Proteinmengen zweier unabhängiger transienter Transfektionsexperimente (in zweifach Ansätzen) bezogen auf das Wildtyp-Konstrukt aufgelistet. Diese Ergebnisse belegen eine deutliche Reduktion der Proteinexpression mit der Verringerung der CpG Dinukleotide und eine deutliche Erhöhung verglichen mit dem Wildtyp-Gen und den kodonoptimierten Genen, korrelierend mit der zusätzlichen Einführung solcher Motive und trotz einer Verschlechterung der Kodonanpassung.

**Tabelle 1: Expressionsvergleich muriner MIP1alpha Gene**

| **Konstrukt** | **SEQ ID NO. ID** | **Expression*** | **StdAbw**** | **Anzahl CpG** | **CAI***** |
|---|---|---|---|---|---|
| pc-maMIP wt | 15 | 100 % | 4 % | 8 | 0,76 |
| pc-maMIP 0 | 5 | 2 % | 9 % | 0 | 0,92 |
| pc-maMIP 2 | 7 | 8 % | 27 % | 2 | 0,93 |
| pc-maMIP 4 | 9 | 7 % | 33 % | 4 | 0,93 |
| pc-maMIP 13 | 11 | 146 % | 5 % | 13 | 0,97 |
| pc-maMIP 42 | 13 | 246 % | 4 % | 42 | 0,72 |

| | | | | | |
|---|---|---|---|---|---|
| * Prozentualer Mittelwert der Proteinmenge aus 2 Versuchen (in Doppelansätzen) im Verhältnis zur Gesamtproteinmenge des Wildtyp-Konstruktes (maMIP wt) ** Standardabweichung *** Codon-Adaptationsindex | | | | | |

### Beispiel 3

### Herstellung von humanen und murinen Cytokingenen mit unterschiedlichem CpG-Gehalt

Um die bisher erhaltenen Ergebnisse und Interpretationen weiter zu festigen, wurden analog zu Beispiel 2 Varianten des humanen MIP1alpha Gens, des humanen GM-CSF Gens, des humanen IL-15 Gens und des murinen GM-CSF-Gens, die sich in der Anzahl an CpG Dinukleotiden vom Wildtyp Gen unterscheiden, künstlich aufgebaut und unter Verwendung der Schnittstellen *Hind*III und *Not*I in den Expressionsvektor pcDNA3.1 subkloniert. Die Bezeichnung der Konstrukte, Anzahl der CpGs sowie die CAI Werte sind in Tabelle 2 wiedergegeben. Die Nukleotid- und Aminosäuresequenzen der Wildtypsequenzen (wt) und der Sequenzen mit veränderter Anzahl an CpG Dinukleotiden sind in SEQ ID NO. 17/18 bis SEQ ID NO. 23/24 und SEQ ID NO. 48/49 bis SEQ ID NO. 54/55 angegeben. Die Expressionskonstrukte wurden mittels Polymerase Kettenreaktion (PCR) unter Verwendung der Oligonukleotide humip-1 und humip-2, hugm-1 und hugm-2, huil-1 und huil-2, magm-1 und magm-2 aus entspechenden cDNA Klonen (von RZPD bezogen) amplifiziert und ebenfalls unter Verwendung der Schnittstellen *Hind*III und *Not*I in den Expressionsvektor pcDNA3.1 einkloniert ("pc-huMIP-wt", GenBank Accession Nummer NM_021006, "pc-huGM-wt", GenBank Accession Nummer M11220, "pc-hulL-wt", GenBank Accession Nummer BC018149, "pc-muGM-wt", GenBank Accession Nummer NM_049969 mit einer Abweichung).

### Überprüfung der Cytokin Expression

Zur Quantifizierung der Cytokin-Expression wurden humane Zellen mit den jeweiligen Expressionskonstrukten transfiziert und die Proteinmenge im Zellkulturüberstand mittels kommerzieller ELISA Testkits gemessen.

Wie in Beispiel 2 beschrieben wurden H1299 Zellen transient mit 15 µg des entsprechenden Expressionsplasmids transfiziert. Der Zellkulturüberstand wurde 48 h nach der Transfektion geerntet. Aus dem Zellkulturüberstand wurden durch Zentrifugation unlösliche Zellbestandteile entfernt.

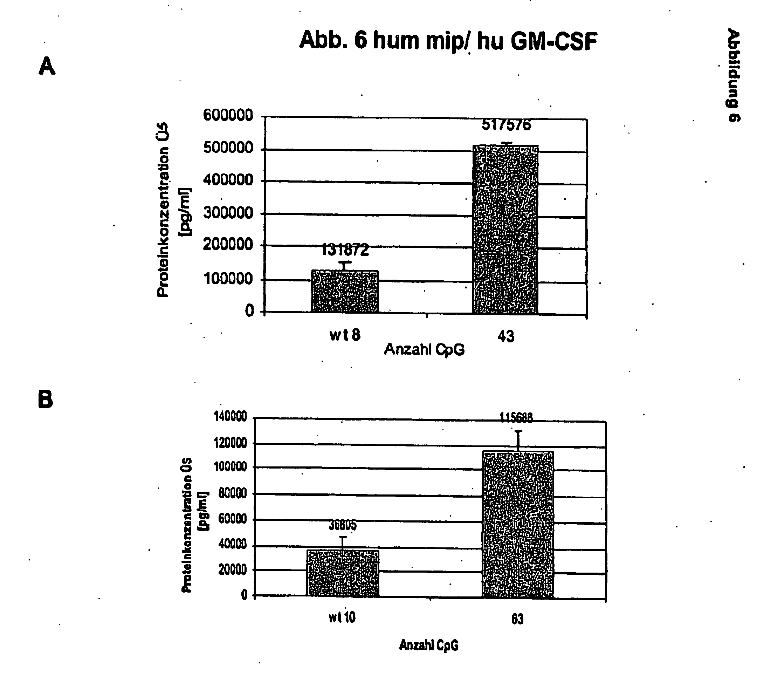

Aus verdünnten Zellkulturüberständen wurde jeweils in einem kommerziell erhältlichen ELISA Assay (R & D Systems für MIP1alpha; BD Pharmingen für GM-CSF und IL-15) die Expression des humanen MIP1alpha, des humanen GM-CSF, humanen des IL-15 und des murinen GM-CSF überprüft. Vergleichbar mit den Daten der erwähnten Expressionskonstrukte, korrelierte die Gesamtmenge an detektierbaren Cytokinen im Kulturüberstand mit der Anzahl an CpGs im Leserahmen. Die Daten sind in Tabelle 2 zusammengefasst. Ein repräsentatives Ergebnis einer Auswertung mittels Cytokin ELISA ist in Abbildung 6 wiedergegeben. Die Balken entsprechen dem Mittelwert zweier unabhängiger Transfektionsansätzen, die Fehlerbalken repräsentieren die jeweilige Standardabweichung. In der Tabelle 2 sind die relativen Proteinmengen jeweils von einem transienten Transfektionsexperiment (in 2-fach Ansätzen) bezogen auf das Wildtyp-Konstrukt aufgelistet. Analog der Ergebnisse aus Beispiel 2 belegen auch diese Ergebnisse eine deutliche Erhöhung der Proteinproduktion, korrelierend mit der zusätzlichen Einführung solcher Motive, verglichen mit den Wildtyp-Genen.

**Tabelle 2: Expressionsvergleich humaner Cytokin-/Chemokingene**

| **Konstrukt** | **SEQ ID NO.** | **Expression*** | **Anzahl CpG** | **CAI**** |
|---|---|---|---|---|
| pc-huMIP wt | 21 | 100 % | 8 | 0,76 |
| pc-huMIP 43 | 17 | 393 % | 43 | 0,72 |
| pc-huGM wt | 23 | 100 % | 10 | 0,82 |
| pc-huGM 63 | 19 | 327 % | 63 | 0,70 |
| pc-hulL wt | 56 | 100 % | 3 | 0,65 |
| pc-huIL 21 | 52 | 313 % | 21 | 0,98 |
| pc-muGM wt | 58 | 100 % | 11 | 0,75 |
| pc-muGM 62 | 54 | 410 % | 62 | 0,75 |

| | | | | |
|---|---|---|---|---|
| * Prozentualer Mittelwert der Proteinmenge aus jeweils einem Versuch, in Doppelansätzen im Verhältnis zur Gesamtproteinmenge des entsprechenden wildtyp Konstruktes (bezeichnet wt) ** Codon-Aadaptationsindex | | | | |

### Beispiel 4

### Herstellung eines Plasmides mit reduzierter Anzahl an CpG Dinukleotiden zur Steigerung der Expression

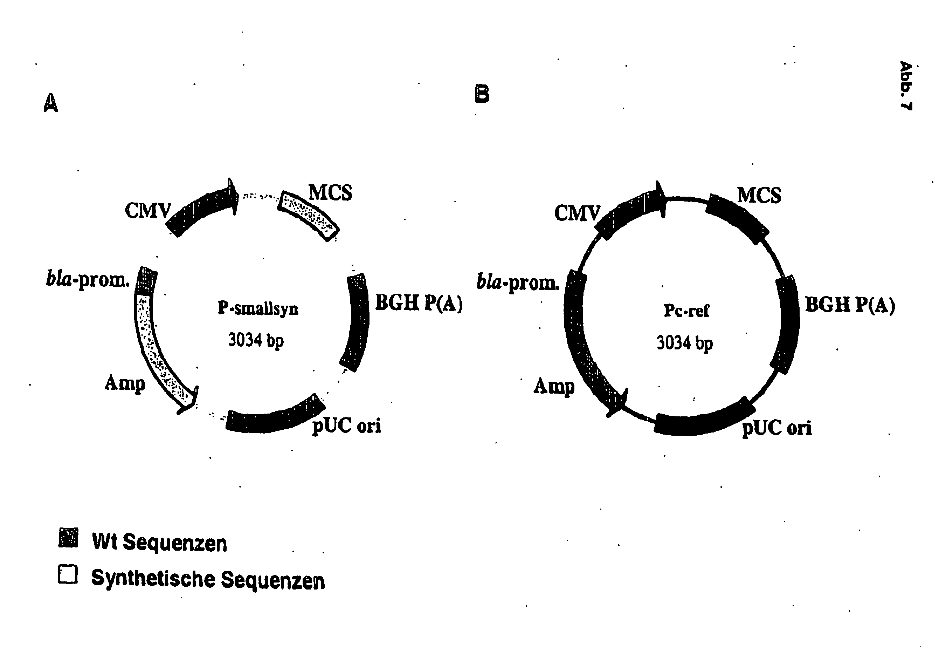

Als Basis für die Herstellung eines modular aufgebauten und in der Anzahl der CpG Dinukleotide soweit als möglich reduzierten Plasmides wurde die Nukleinsäuresequenz des Plasmides pcDNA5 (Invitrogen) herangezogen. Die DNA Sequenz, die für das Ampicillinresistenzgen (bla) kodiert wurde wie unter Beispiel 1 beschrieben synthetisch hergestellt, und unter Verwendung der Restriktionsschnittstellen *Cla*I und *Bgl*II subkloniert. Die Anzahl CpGs wurde dabei von 72 auf 2 reduziert. Ebenso wurde die *Multiple Cloning Site* neu konzipiert, synthetisch aufgebaut und unter Verwendung der Restriktionsschnittstellen SacI und *Pme*I subkloniert, wodurch die Anzahl der CpGs von 11 auf 1 verringert wurde. Der CMV Promotor (31 CpGs), die BGH Polyadenylierungsstelle (3 CpGs) und der pUC Replikationsursprung (45 CpGs) wurden unverändert in das Plasmid integriert. Die Hygromycin-Resistenzkassete wurde deletiert. Der CMV Promotor wurde durch PCR Amplifikation mit den Oligonukleotiden CMV-1 und CMV-2, die zusätzlich eine *Cla*I und eine *Sac*I Restriktionsschnitstelle 3' bzw. 5' anfügten, kloniert. Analog wurde der pUC ori mit den Oligonukleotiden ori-1 (enthält *Xm*al Schnittstelle) und ori-2 (enthält *Bgl*II Schnittstelle) und die BGH Polyadenylierungsstelle mit den Oligonukleotiden pa-1 (*Pme*I) und pa-2 (*Xma*I) durch PCR amplifiziert und unter Verwendung der entsprechenden Restriktionsenzyme subkloniert. Das Plasmid pcDNA5 wurde als Template bei allen PCR Reaktionen eingesetzt. Der Aufbau dieses Plasmides ist schematisch in Abbildung 7A ("P-smallsyn") dargestellt und die vollständige Sequenz ist in SEQ ID NO. 25 angegeben.

Um den Einfluss der Anzahl von CpGs im Vektor auf die Expressionshöhe eines Transkriptes zu untersuchen, wurde der Referenzvektor so modifiziert, dass er als Kontrolle verwendet werden konnte. Durch PCR Amplifikation unter Verwendung der Oligonukleotide ref-del-1 und ref-del-2, die jeweils eine Nsil Restriktionsschnittstelle am 5' Ende einfügten, Spaltung mit NsiI und Ligation wurde die Hygromycin-Resistenzkassette aus dem Plasmid pcDNA5 entfernt (siehe Abbildung 6B, "Pc-ref").

Als Testtranskript wurde das von HIV-1 abgeleitete p24 Kapsidprotein verwendet. Die kodierende Region des bereits früher auf eine Expression in humanen Zellen optimierte p24 (Graf et al. 2000) wurde mittels PCR unter Verwendung der Oligonukleotide p24-1 und p24-2 aus einem HIV-1 syngag Konstrukt (Graf et al., 2000) amplifiziert und unter Verwendung der Schnittstellen *Hind*III und *Bam* HI in die beiden Vergleichsvektoren einkloniert ("R/p24" und "s/p24").

### Überprüfung der HIV-1 p24 Expression in unterschiedlichem Vektorhintergrund

Um den Einfluss der CpG Anzahl im Vektor aus die Expression des Transkriptes zu überprüfen, wurden die Konstrukte R/p24 und s/p24 transient in humane Zellen transfiziert und die Expression des p24 analysiert.

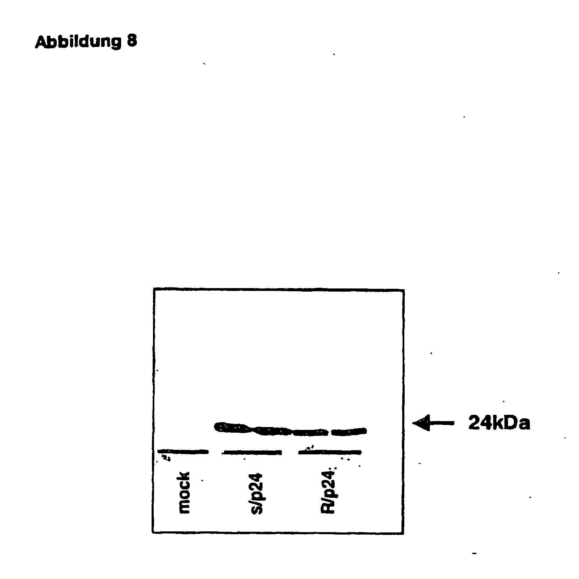

Wie in Beispiel 2 beschrieben, wurden H1299 Zellen transient mit 15 µg des entsprechenden Expressionsplasmids transfiziert. Zellen wurden 48 h nach der Transfektion geerntet. Die transfizierten Zellen wurden wie im Beispiel 1 beschrieben lysiert und die Gesamtproteinmenge des Überstandes wurde mit dem Bio-Rad Protein Assay bestimmt. 50 µg Gesamtprotein aus Zellysaten wurden wie in Beispiel 1 beschrieben in einer Western Blot Analyse mit einem monoklonalen p24-spezifischen Antikörper 13-5 (Wolf et al., 1990) auf die Expression des p24 überprüft (Abb. 8). In zwei unabhängigen Transfektionsansätzen war eine deutlich höhere p24 Expression nach Transfektion des smallsyn Konstruktes (s/p24) zu erkennen.

### Herstellung von HIV p24 Genen mit unterschiedlichem CpG-Gehalt

Zwei Varianten des von HIV-1 abgeleiteten Kapsidproteigens p24, die sich in der Anzahl an CpG Dinukleotiden unterscheiden, wurden hergestellt. Das syn p24 Gen wies 38 CpGs auf, das p24ΔCpG Gen hatte keine CpGs. Das CpG depletierte Gen p24ΔCpG wurde wie unter Beispiel 1 beschrieben künstlich aufgebaut und unter Verwendung der Schnittstellen *Hind*III und *Bam* HI in den Expressionsvektor P-smallsyn (in Beispiel 4 beschrieben) ("s/p24ΔCpG") und in den Referenzvektor Pc-ref ("R/p24ΔCpG") kloniert. Die Nukleotid- und Aminosäuresequenzen von p24ΔCpG ist in SEQ ID NO. 26/27 angegeben. Als Referenzkonstrukte wurden die Plasmide R/p24 und s/p24, die im Beispiel 4 beschrieben sind, verwendet.

### Überprüfung der HIV-1 p24 Expression

Um den Einfluss der CpG Anzahl im Vektor und im insert (Transkript) zu überprüfen, wurden die Konstrukte R/p24, R/p24ΔCpG, s/p24 und s/p24ΔCpG transient in humane Zellen transfiziert und die Expression des p24 analysiert.

Wie in Beispiel 2 beschrieben, wurden H1299 Zellen transient mit 15 µg des entsprechenden Expressionsplasmids transfiziert. Zellen wurden 48 h nach der Transfektion geerntet. Die transfizierten Zellen wurden wie in Beispiel 1 beschrieben lysiert und die Gesamtproteinmenge des Lysates wurde mit dem Bio-Rad Protein Assay bestimmt.

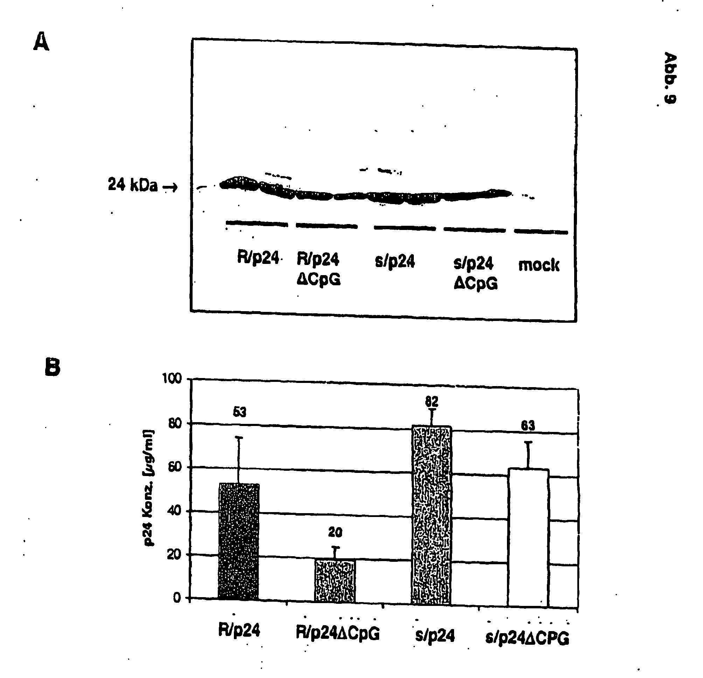

50 µg Gesamtprotein aus Zellysaten wurden wie in Beispiel 1 beschrieben in einer Western Blot Analyse mit einem monoklonalen p²⁴-spezifischen Antikörper 13-5 auf die Expression des p24 überprüft (Abb. 9A). Wie in Beispiel 4 bereits gezeigt, führte die Verwendung des CpG deletierten Vektors P-smallsyn bei identischem Transgen zu einer sichtbaren Steigerung der p24 Produktion (Vergleich R/p24 und s/p24). Vergleichbar mit den Daten der GFP- und der Cytokin/Chemokin-Expressionskonstrukten, korrelierte die Menge an detektierbaren p24 im Zelllysat, bei Verwendung des identischen Vektorhintergrundes, mit der Anzahl an CpGs im Leserahmen (Vergleich R/p24 und R/-p24ΔCpG sowie s/p24 und s/p24ΔCpG). Die Daten wurden in einem p24-spezifischen ELISA bestätigt (Abb. 9B). Das Konstrukt mit 38 CpGs (p24) wies eine ca. 2,5 fach (Pc-ref) bzw. ca 25 % (P/smallsyn) höhere Menge an p24 auf als das Konstrukt ohne CpGs (p24 DcpG). Die Ergebnisse sind in Abb. 9 gezeigt.

Die Korrelation der Proteinproduktion mit der Anzahl an CpG Dinukleotiden konnte in den hier genannten Beispielen belegt werden. Die ausgewählten Gene stammen aus so unterschiedlichen Organismen wie einer Qualle, einem humanpathogenen Virus und Säugetieren. Es liegt daher nahe, diesen Mechanismus als allgemeingültig anzusehen. Die Beispiele belegen weiterhin, dass diese Korrelation *in vitro* sowohl bei einer transienten Transfektion, als auch bei stabilen rekombinanten Zellen Gültigkeit hat. Das hier beschriebene Verfahren, die Genexpression in Eukaryonten durch gezielte Modulation der CpG Dinukleotide, sowohl in der kodierenden Region, als auch im Vektorhintergrund, gezielt zu verändern, kann folglich für die Produktion von Biomolekülen für biotechnologische, diagnostische oder medizinische Anwendung verwendet werden.

### Beschreibung der Sequenzen

### 1. Oligonukleotide

| SEQ ID NO. | Bezeichnung | Sequenz 5' - 3' |
|---|---|---|
| 28 | huGFP-1 | |
| 29 | huGFP-2 | AGTAGGATCCTATTACTTGTACAGCTCGT |
| 30 | RT-oligo1 | CCCTGAAGTTCATCTGCACC |
| 31 | RT-oligo2 | GATCTTGAAGTTCACCTTGATG |
| 32 | mamip-1 | CAGGTACCAAGCTTATGAAGGTCTCCACCACTGC |
| 33 | mamip-2 | |
| 34 | hugm-1 | CAGGTACCAAGCTTATGTGGCTGCAGAGCCTGC |
| 35 | hugm-2 | |
| 36 | humip-1 | CAGTACCAAGCTTATGCAGGTCTCCACTGCTGC |
| 37 | humip-2 | |
| 38 | p24-1 | |
| 39 | p24-2 | |
| 40 | CMV-1 | |
| 41 | CMV-2 | GAATGAGCTCTGCTTATATAGACC |
| 42 | ori-1 | |
| 43 | ori-2 | |
| 44 | pa-1 | |
| 45 | pa-2 | |
| 46 | ref-del-1 | |
| 47 | ref-del-2 | AGTCATGCATCCATAGAGCCCACCGCATCCCCA |
| 48 | huil-1 | CAGGTACCAAGCTTATGAGAATTTCGAAACCAC |
| 49 | huil-2 | |
| 50 | magm-1 | |
| 51 | magm-2 | |

### 2. Polypeptid-kodierende Sequenzen und Vektorsequenzen

### Referenzen

Akiyama, Y., Maesawa, C., Ogasawara, S., Terashima, M. and Masuda, T. (2003) Cell-type-specific repression of the maspin gene is disrupted frequently by demethylation at the promoter region in gastric intestinal metaplasia and cancer cells, Am.J.Pathol. 163, 1911-1919.
Antequera, F. and Bird, A. (1993) Number of CpG islands and genes in human and mouse, Proc.Natl.Acad.Sci.U.S.A 90, 11995-11999.
Ausubel, F. M., Brent, R., Kingston, R. E., Moore, d. d., Seidman, J. G., Smith, J. A. and Struhl, K. (1994) Percentage of Kodon Synonomous Usage and Frequency of Kodon Occurrence in Various Organisms, Current Protocols in Molecular Biology 2, A1.8-A1.9.
Bird, A. P. (1980) DNA methylation and the frequency of CpG in animal DNA, Nucleic Acids Res. 8, 1499-1504.
Chevalier-Mariette, C., Henry, I., Montfort, L., Capgras, S., Forlani, S., Muschler, J. and Nicolas, J. F. (2003) CpG content affects gene silencing in mice: evidence from novel transgenes, Genome Biol. 4, R53.
Choi, Y. S., Kim, S., Kyu, L. H., Lee, K. U. and Pak, Y. K. (2004) In vitro methylation of nuclear respiratory factor-1 binding site suppresses the promoter activity of mitochondrial transcription factor A, Biochem.Biophys.Res.Commun. 314, 118-122.
Deml L., Bojak A., Steck S., Graf M., Wild J., Schirmbeck R., Wolf H., Wagner R. (2003) Multiple Effects of Codon Usage Optimization on Expression and Immunognicity of DNA Candidate Vaccines Encoding the Human Immunodeficiency Virus Type 1 Gag Gene, J. Virol. 75 No. 22, 10999-11001.
Deng, G., Chen, A., Pong, E. and Kim, Y. S. (2001) Methylation in hMLH1 promoter interferes with its binding to transcription factor CBF and inhibits gene expression, Oncogene 20, 7120-7127.
Duan J. und Antezana A. (2003) Mammalian Mutation Pressure, Synonymous Codon Choice, and mRNA Degradation, J. Mol. Evol. 57, 694-701
Hendrich, B. and Bird, A. (1998) Identification and characterization of a family of mammalian methyl-CpG binding proteins, Mol.Cell Biol. 18, 6538-6547.
Hisano, M., Ohta, H., Nishimune, Y. and Nozaki, M. (2003) Methylation of CpG dinucleotides in the open reading frame of a testicular germ cellspecific intronless gene, Tact1/Actl7b, represses its expression in somatic cells, Nucleic Acids Res. 31, 4797-4804.
Hsieh, C. L. (1994) Dependence of transcriptional repression on CpG methylation density, Mol.Cell Biol. 14, 5487-5494.
Ivanova, T., Vinokurova, S., Petrenko, A., Eshilev, E., Solovyova, N., Kisseljov, F. and Kisseljova, N. (2004) Frequent hypermethylation of 5' flanking region of TIMP-2 gene in cervical cancer, Int.J.Cancer 108, 882-886.
Jones, P. L., Veenstra, G. J., Wade, P. A., Vermaak, D., Kass, S. U., Landsberger, N., Strouboulis, J. and Wolffe, A. P. (1998) Methylated DNA and MeCP2 recruit histone deacetylase to repress transcription, Nat.Genet. 19, 187-191.
Kang, G. H., Lee, S., Lee, H. J. and Hwang, K. S. (2004) Aberrant CpG island hypermethylation of multiple genes in prostate cancer and prostatic intraepithelial neoplasia, J.Pathol. 202, 233-240.
Kudo, S. (1998) Methyl-CpG-binding protein MeCP2 represses Sp1-activated transcription of the human leukosialin gene when the promoter is methylated, Mol.Cell Biol. 18, 5492-5499.
Laemmli, U. K. (1970) Cleavage of structural proteins during the assembly of the head of bacteriophage T4, Nature 227, 680-685.
Larsen, F., Gundersen, G., Lopez, R. and Prydz, H. (1992) CpG islands as gene markers in the human genome, Genomics 13, 1095-1107.
Li, Q. L., Kim, H. R., Kim, W. J., Choi, J. K., Lee, Y. H., Kim, H. M., Li, L. S., Kim, H., Chang, J., Ito, Y., Youl, L. K. and Bae, S. C. (2004) Transcriptional silencing of the RUNX3 gene by CpG hypermethylation is associated with lung cancer, Biochem.Biophys.Res.Commun. 314, 223-228.
Nan, X., Ng, H. H., Johnson, C. A., Laherty, C. D., Turner, B. M., Eisenman, R. N. and Bird, A. (1998) Transcriptional repression by the methyl-CpG-binding protein MeCP2 involves a histone deacetylase complex, Nature 393, 386-389.
Shen, J. C., Rideout, W. M., III and Jones, P. A. (1994) The rate of hydrolytic deamination of 5-methylcytosine in double-stranded DNA, Nucleic Acids Res. 22, 972-976.
Sved, J. and Bird, A. (1990) The expected equilibrium of the CpG dinucleotide in vertebrate genomes under a mutation model, Proc.Natl.Acad.Sci.U.S.A 87, 4692-4696.
Takai, D. and Jones, P. A. (2002) Comprehensive analysis of CpG islands in human chromosomes 21 and 22, Proc.Natl.Acad.Sci.U.S.A 99, 3740-3745.
Voo, K.S., Carlone, D.L., Jacobsen, B.U., Flodin, A., und Skalnik, D. (2000) Cloning of a Mammalian Transcriptional Activator That Binds Unmethylated CpG motifs and Shares a CXXC Domain with DNA Mathyltransferase, Human Trithorax, and Methyl-CpG Binding Domain Protein I, Mol. And Cell. Biol. Mar. 2000, 2108-2121.
Wade, P. A., Gegonne, A., Jones, P. L., Ballestar, E., Aubry, F. and Wolffe, A. P. (1999) Mi-2 complex couples DNA methylation to chromatin remodelling and histone deacetylation, Nat.Genet. 23, 62-66.
Wise, T. L. and Pravtcheva, D. D. (1999) The undermethylated state of a CpG island region in igf2 transgenes is dependent on the H19 enhancers, Genomics 60, 258-271.
Wolf, H., Modrow, S., Soutschek, E., Motz, M., Grunow, R. and Döbl, H. (1990) Production, mapping and biological characterisation of monoclonal antibodies to the core protein (p24) of the human immunodeficiency virus type 1., AIFO 1, 24-29.
Wu, Q., Shi, H., Suo, Z. and Nesland, J. M. (2003) 5'-CpG island methylation of the FHIT gene is associated with reduced protein expression and higher clinical stage in cervical carcinomas, Ultrastruct.Pathol. 27, 417-422.
Yao, X., Hu, J. F., Daniels, M., Shiran, H., Zhou, X., Yan, H., Lu, H., Zeng, Z., Wang, Q., Li, T. and Hoffman, A. R. (2003) A methylated oligonucleotide inhibits IGF2 expression and enhances survival in a model of hepatocellular carcinoma, J.Clin.lnvest 111, 265-273.
Yoshida, M., Nosaka, K., Yasunaga, J. I., Nishikata, I., Morishita, K. and Matsuoka, M. (2003) Aberrant expression of the MEL1S gene identified in association with hypomethylation in adult T-cell leukemia cells, Blood.

## Patentansprüche

1. Verfahren zur gezielten Modulation der Genexpression, umfassend:
(i) Bereitstellen einer zu exprimierenden Zielnukleinsäuresequenz,
(ii) Modifizieren der Zielnukleinsäuresequenz, wobei die Anzahl an in der Zielnukleinsäuresequenz vorhandenen CpG-Dinukleotiden unter Verwendung der Degeneration des genetischen Codes zur Erhöhung der Genexpression erhöht wird, wobei die Zielnukleinsäuresequenz so modifiziert ist, dass sie in Bezug auf das verwendete Expressionssystem kodonoptimiert ist und dass die Anzahl der CpG-Dinukleotide im Vergleich mit der kodonoptimierten, von der Wildtyp-Sequenz abgeleiteten Sequenz unter Verwendung der Degeneration des genetischen Codes erhöht ist,
(iii) Klonieren der so modifizierten Zielnukleinsäuresequenz mit modifizierter Anzahl der CpG-Dinukleotide in einen geeigneten Expressionsvektor in operativer Verknüpfung mit einer geeigneten Transkriptionskontrollsequenz,
(iv) Exprimieren der modifizierten Zielnukleinsäuresequenz in einem geeigneten Säuger-Expressionssystem,
wobei das Verfahren kein Verfahren zur therapeutischen Behandlung des menschlichen oder tierischen Körpers ist.

2. Verfahren nach Anspruch 1, wobei in Schritt (ii) das Modifizieren der Zielnukleinsäuresequenz so durchgeführt wird, dass neben der Erhöhung der Anzahl der CpG-Dinukleotide eine oder mehrere zusätzliche Modifikationen auf Nukleinsäureebene vorgenommen werden.

3. Verfahren nach Anspruch 2, wobei die zusätzlichen Modifikationen auf Nukleinsäureebene ausgewählt werden aus der Gruppe bestehend aus: Einfügen oder Eliminieren von die RNA-Sekundärstruktur stabilisierenden Sequenzabfolgen, Regionen mit erhöhter Selbsthomologie, Regionen mit erhöhter Homologie zum natürlichen Gen, RNA-Instabilitätsmotiven, Splice-aktivierenden Motiven, Polyadenylierungs-Motiven, andeninreichen Motiven, Endonukleasen-Erkennungsstellen.

4. Verfahren nach einem der Ansprüche 1 bis 3, wobei die Genexpression erhöht wird.

5. Verfahren nach einem der vorhergehenden Ansprüche, wobei die zu exprimierende Zielnukleinsäuresequenz zu dem Säuger-Expressionssystem heterolog ist.

6. Verfahren nach einem der vorhergehenden Ansprüche, wobei als Säuger-Expressionssystem eine Zelle, ausgewählt aus der Gruppe bestehend aus Säugerzellen, Humanzellen und somatischen Zellen; oder eine zellfreie Expressionsumgebung verwendet wird.

7. Verfahren nach einem der vorhergehenden Ansprüche, wobei die zu exprimierende Zielnukleinsäuresequenz eukaryontischen, prokaryontischen, viralen oder synthetischen Ursprungs ist.

8. Verfahren nach einem der vorhergehenden Ansprüche, wobei als Expressionssystem ein methylierungsarmes System verwendet wird.

9. Verfahren nach einem der vorhergehenden Ansprüche wobei die modifizierte Zielnukleinsäuresequenz und die Transkriptionskonttollsequenz nicht mit CpG-Inseln assoziiert wird.

10. Verfahren nach einem der vorhergehenden Ansprüche, wobei die Anzahl der CpG-Dinukleotide um mindestens zwei erhöht wird.

11. Verfahren nach einem der vorhergehenden Ansprüche, wobei die Anzahl der CpG-Dinukleotide um mindestens 10 %, vorzugsweise mindestens 50 %, vorzugsweise mindestens 100% erhöht wird.

12. Verfahren nach Anspruch einem der vorhergehenden Ansprüche, wobei die Zielnukleinsäuresequenz für eine RNA, Derivate oder Mimetika davon, ein Peptid oder Polypeptid, ein modifiziertes Peptid oder Polypeptid, ein Protein oder ein modifiziertes Protein kodiert.

13. Verfahren nach Anspruch 12, wobei die Zielnukleinsäuresequenz für ein therapeutisches und/oder diagnostisches Protein kodiert.

14. Verfahren nach Anspruch 13, wobei die Zielnukleinsäuresequenz für ein Protein, ausgewählt aus humanen, parasitären, viralen oder bakteriellen Proteinen, Enzymen, Hormonen, Impfstoffen, Botenstoffen und Regulatorproteinen, kodiert.

15. Verfahren nach Anspruch 13 oder 14, wobei die Zielnukleinsäuresequenz für ein Protein ausgewählt aus Asparaginase, Adenosin Deaminase, Insulin, tPA, Gerinnungsfaktoren, Vitamin L-Epoxid Reduktase, Erythropoietin, Follikelstimuliereadem Hormon, Östrogenen, knochenmorphogenetischen Proteinen, Antithrombin, HIV-, HBV-, HCV-, Intluenza-, Borrelien-, Haemophilus-, Menigococcus-, Anthrax-abgeleiteten, Botulinus Toxoid, Diphterie Toxoid, Tetanus Toxoid, Plasmodium-Proteinen, Blutgruppenantigenen, HLA-Proteinen, Cytokinen, Chemokinen, G-CSF, GM-CSF, Interleukinen, Interferonen, PDGF, TNF, RANTES, MIP1α und Transkriptionsfaktoren, kodiert.

16. Verfahren nach Anspruch 12, wobei die Zielnukleinsäuresequenz für eine funktionelle RNA kodiert.

17. Modifizierte Nukleinsäure mit einer transküptionsfähigen Region, die in einem Säuger-Expressionssystem exprimiert werden kann, und die von einer Wildtyp-Sequenz abgeleitet ist,
wobei die transkriptionsfähige Region so modifiziert ist, dass sie in Bezug auf das verwendete Säuger-Expressionssystem kodonoptimiert ist, wobei eine Kodonwahl verwendet wird, wie sie in Säugerzellen am häufigsten oder zweithiäufigsten verwendet wird,
und dass die Anzahl an CpG-Dinukleotiden im Vergleich mit der kodonoptimierten, von der Wildtyp-Sequenz abgeleiteten Sequenz unter Verwendung der Degeneration des genetischen Codes erhöht ist,
wobei die Anzahl der CpG-Dinukleotide im Vergleich mit der Wildtyp-Sequenz um mindestens das Fünffache, vorzugsweise um das Zehnfache oder mehr erhöht ist.

18. Nukleinsäure nach Anspruch 17, wobei die Nukleinsäure nicht mit einer CpG-Insel assoziiert ist.

19. Vektor, umfassend eine Nukleinsäure nach einem der Ansprüche 17 bis 18 in operativer Verknüpfung mit einer geeigneten Transkriptionskontrollsequenz.

20. Vektor nach Anspruch 19, wobei die Transkriptionskontrollsequenz einen Promotor umfasst.

21. Vektor nach Anspruch 20, wobei der Promotor ein konstitutiv aktiver Promotor ist.

22. Vektor nach Anspruch 21, wobei der konstitutiv aktive Promotor ausgewählt ist aus (Cytomegalovirus) CMV Promotor und Simian Virus 40 (SV40) Promotor.

23. Vektor nach Anspruch 20, wobei der Promotor ein induzierbarer Promotor ist.

24. Vektor nach Anspruch 23, wobei der induzierbare Promotor ein Tetraryclin-abhängiger Promotor ist.

25. Vektor nach einem der Ansprüche 19 bis 24, wobei der Promotor nicht mit einer CpG-Insel assoziiert ist.

26. Vektor nach einem der Ansprüche 19 bis 25, wobei die von der Nukleinsäure nach einem der Ansprüche 17 bis 18 verschiedenen, auf dem Vektor vorhandenen Sequenzen oder Teile davon eine verringerte Anzahl an CpG-Dinukleotiden aufweisen.

27. Vektor nach einem der Ansprüche 19 bis 26, wobei die von der Nukleinsäure nach einem der Ansprüche 17 bis 18 verschiedenen Sequenzen oder Teile davon eine um etwa 25 %, vorzugsweise 50 %, stärker bevorzugt 75 %, stärker bevorzugt um 100% verringerte Anzahl an CpG-Dinukleotiden aufweisen.

28. Vektor nach einem der Ansprüche 20 bis 27 mit der in SEQ ID NO. 25 dargestellten Nukleinsäuresequenz.

29. Zelle, enthaltend eine Nukleinsäure oder einen Vektor nach einem der Ansprüche 17 bis 28.

30. Arzneimittel, umfassend als Wirkstoff einen Vektor und/oder eine Zelle nach einem der Ansprüche 19 bis 29.

31. Verwendung einer Nukleinsäure und/oder eines Vektors und/oder einer Zelle nach einem der Ansprüche 18 bis 29 zur Herstellung von Impfstoffen.

32. Nukleinsäure mit einer Sequenz ausgewählt aus SEQ ID NO. 1, 5, 7, 9 und 26.

## Claims

1. Method for the targeted modulation of gene expression, comprising:
(i) provision of a target nucleic acid sequence to be expressed,
(ii) modification of the target nucleic acid sequence, the number of CpG dinucleotides present in the target nucleic acid sequence being increased using the degeneracy of the genetic code to increase gene expression, wherein the target nucleic acid sequence is modified such that it is codon-optimized in relation to the expression system used and such that the number of CpG dinucleotides is increased compared to the codon-optimized sequence derived from the wild-type sequence using the degeneracy of the genetic code,
(iii) cloning of the thereby modified target nucleic acid sequence with a modified number of CpG dinucleotides into a suitable expression vector in operative linkage with a suitable transcriptional control sequence,
(iv) expression of the modified target nucleic acid sequence in a suitable mammalian expression system,
wherein said method is not a method for treatment of the human or animal body by therapy.

2. Method according to claim 1, wherein in step (ii) the modification of the target nucleic acid sequence is carried out in such a manner that, in addition to increasing the number of CpG dinucleotides, one or more additional modifications is/are carried out at the nucleic acid level.

3. Method according to claim 2, wherein the additional modifications at the nucleic acid level are selected from the group consisting of: insertion or elimination of sequence orders stabilizing the RNA secondary structure, regions with elevated self-homology, regions with elevated homology to the natural gene, RNA-instability motifs, splice-activating motifs, polyadenylation motifs, adenine-rich motifs, endonuclease recognition sites.

4. Method according to one of claims 1 to 3, wherein gene expression is increased.

5. Method according to one of the preceding claims, wherein the target nucleic acid sequence to be expressed is heterologous to the mammalian expression system.

6. Method according to one of the preceding claims, wherein a cell selected from the group consisting of mammalian cells, human cells and somatic cells; or a cell-free expression environment is used as the mammalian expression system.

7. Method according to one of the preceding claims, wherein the target nucleic acid sequence to be expressed is of eukaryotic, prokaryotic, viral or synthetic origin.

8. Method according to one of the preceding claims, wherein a low methylation system is used as the expression system.

9. Method according to one of the preceding claims, wherein the modified target nucleic acid sequence and the transcriptional control sequence are not associated with CpG islands.

10. Method according to one of the preceding claims, wherein the number of CpG dinucleotides is increased by at least two.

11. Method according to one of the preceding claims, wherein the number of CpG dinucleotides is increased by at least 10%, preferably at least 50%, preferably at least 100%.

12. Method according to one of the preceding claims, wherein the target nucleic acid sequence encodes an RNA, derivates or mimetics thereof, a peptide or polypeptide, a modified peptide or polypeptide, a protein or a modified protein.

13. Method according to claim 12, wherein the target nucleic acid sequence encodes a therapeutic and/or diagnostic protein.

14. Method according to claim 13, wherein the target nucleic acid sequence encodes a protein selected from human, parasitic, viral or bacterial proteins, enzymes, hormones, vaccines, messenger substances and regulatory proteins.

15. Method according to claim 13 or 14, wherein the target nucleic acid sequence encodes a protein selected from asparaginase, adenosine deaminase, insulin, tPA, clotting factors, vitamin L epoxide reductase, erythropoietin, follicle-stimulating hormone, oestrogens, bone morphogenetic proteins, antithrombin, HIV-, HBV-, HCV-, influenza-, borrelia-, haemophilus-, meningococcus-, anthrax-derived proteins, botulinus toxoid, diphtheria toxoid, tetanus toxoid, plasmodium proteins, blood group antigens, HLA proteins, cytokines, chemokines, G-CSF, GM-CSF, interleukins, interferons, PDGF, TNF, RANTES, MIP1α and transcription factors.

16. Method according to claim 12, wherein the target nucleic acid sequence encodes a functional RNA.

17. Modified nucleic acid with a region capable of transcription that can be expressed in a mammalian expression system, and which is derived from a wild-type sequence, wherein the region capable of transcription is modified such that it is codon-optimized in relation to the mammalian expression system used, wherein a codon choice is used as is most or second-most commonly used in mammalian cells, and such that the number of CpG dinucleotides is increased compared to the codon-optimized sequence derived from the wild-type sequence using the degeneracy of the genetic code, wherein the number of CpG dinucleotides is increased compared to the wild-type sequence by at least a factor of five, preferably by a factor of ten or more.

18. Nucleic acid according to claim 17, wherein the nucleic acid is not associated with a CpG island.

19. Vector comprising a nucleic acid according to one of claims 17 to 18 in operative linkage with a suitable transcriptional control sequence.

20. Vector according to claim 19, wherein the transcriptional control sequence comprises a promoter.

21. Vector according to claim 20, wherein the promoter is a constitutively active promoter.

22. Vector according to claim 21, wherein the constitutively active promoter is selected from (cytomegalovirus) CMV promoter and simian virus 40 (SV40) promoter.

23. Vector according to claim 20, wherein the promoter is an inducible promoter.

24. Vector according to claim 23, wherein the inducible promoter is a tetracycline-dependent promoter.

25. Vector according to one of claims 19 to 24, wherein the promoter is not associated with a CpG island.

26. Vector according to one of claims 19 to 25, wherein the sequences or parts thereof present on the vector and different from the nucleic acid according to one of claims 17 to 18 have a reduced number of CpG dinucleotides.

27. Vector according to one of claims 19 to 26, wherein the sequences or parts thereof different from the nucleic acid according to one of claims 17 to 18 have a number of CpG dinucleotides reduced by about 25%, preferably 50%, more preferably 75%, more preferably by 100%.

28. Vector according to one of claims 20 to 27, with the nucleic acid sequence shown in SEQ ID No. 25.

29. Cell containing a nucleic acid or a vector according to one of claims 17 to 28.

30. Medicament comprising as active substance a nucleic acid and/or a vector and/or a cell according to one of claims 19 to 29.

31. Use of a nucleic acid and/or a vector and/or a cell according to one of claims 18 to 29 for the production of vaccines.

32. Nucleic acid with a sequence selected from SEQ ID Nos. 1, 5, 7, 9 and 26.

## Revendications

1. Procédé pour la modulation ciblée de l'expression génique comprenant :
(i) la fourniture d'une séquence d'acide nucléique cible destinée à être exprimée,
(ii) la modification de la séquence d'acide nucléique cible, où le nombre de dinucléotides CpG présents dans la séquence nucléotidique cible est augmenté au moyen de la dégénérescence du code génétique pour augmenter l'expression génique, où la séquence d'acide nucléique cible est modifiée de telle manière qu'elle est optimisée quant aux codons par rapport au système d'expression utilisé et que le nombre des dinucléotides CpG est augmenté par rapport à une séquence dérivée de la séquence de type sauvage, optimisée quant aux codons, au moyen de la dégénérescence du code génétique,
(iii) le clonage de la séquence d'acide nucléique cible à nombre de dinucléotides CpG modifié ainsi modifiée dans un vecteur d'expression approprié en liaison fonctionnelle avec une séquence de contrôle de la transcription appropriée,
(iv) l'expression de la séquence d'acide nucléique cible modifiée dans un système d'expression de mammifère approprié,
où le procédé n'est pas un procédé de traitement thérapeutique de l'organisme humain ou animal.

2. Procédé selon la revendication 1 où, dans l'étape (ii), la modification de la séquence d'acide nucléique cible est réalisée de telle manière qu'outre l'augmentation du nombre des dinucléotides CpG, une ou plusieurs modifications supplémentaires sont réalisées au niveau de l'acide nucléique.

3. Procédé selon la revendication 2 où les modifications supplémentaires au niveau de l'acide nucléique sont choisies dans le groupe consistant en : insertion ou élimination de suites de séquence stabilisant la structure secondaire de l'ARN, de régions à autohomologie augmentée, de régions à homologie avec le gène naturel augmentée, de motifs d'instabilité de l'ARN, de motifs activant l'épissage, de motifs de polyadénylation, de motifs riches en adénine, de sites de reconnaissance pour des endonucléases.

4. Procédé selon l'une des revendications 1 à 3, où l'expression génique est augmentée.

5. Procédé selon l'une des revendications précédentes où la séquence d'acide nucléique cible destinée à être exprimée est hétérologue pour le système d'expression de mammifère.

6. Procédé selon l'une des revendications précédentes où une cellule choisie dans le groupe consistant en les cellules de mammifère, les cellules humaines et les cellules somatiques, ou un environnement d'expression sans cellules, est utilisé(e) comme système d'expression de mammifère.

7. Procédé selon l'une des revendications précédentes où la séquence d'acide nucléique cible destinée à être exprimée est d'origine eucaryote, procaryote, virale ou synthétique.

8. Procédé selon l'une des revendications précédentes où un système pauvre en méthylation est utilisé comme système d'expression.

9. Procédé selon l'une des revendications précédentes où la séquence d'acide nucléique cible modifiée et la séquence de contrôle de la transcription ne sont pas associées à des îlots de CpG.

10. Procédé selon l'une des revendications précédentes où le nombre des dinucléotides CpG est augmenté d'au moins deux.

11. Procédé selon l'une des revendications précédentes où le nombre des dinucléotides CpG est augmenté d'au moins 10 %, de préférence d'au moins 50 %, de préférence d'au moins 100 %.

12. Procédé selon l'une des revendications précédentes où la séquence d'acide nucléique cible code un ARN, des dérivés ou numétiques de celui-ci, un peptide ou un polypeptide, un peptide ou un polypeptide modifié, une protéine ou une protéine modifiée.

13. Procédé selon la revendication 12 où la séquence d'acide nucléique cible code une protéine thérapeutique et/ou diagnostique.

14. Procédé selon la revendication 13 où la séquence d'acide nucléique cible code une protéine choisie parmi les protéines humaines, parasitaires, virales ou bactériennes, les enzymes, les hormones, les vaccins, les messagers et les protéines régulatrices.

15. Procédé selon la revendication 13 ou 14 où la séquence d'acide nucléique cible code une protéine choisie parmi l'asparaginase, l'adénosine désaminase, l'insuline, le tPA, les facteurs de coagulation, la vitamine L-époxyde réductase, l'érythropoïétine, la folliculostimuline, les estrogènes, les protéines morphogénétiques osseuses, l'antithrombine, les protéines dérivées de VIH, VHB, VHC, influenza, Borrelia, Haemophilus, Meningococcus, Anthrax, l'anatoxine botulique, l'anatoxine diphtérique, l'anatoxine tétanique, les protéines de Plasmodium, les antigènes de groupes sanguins, les protéines de HLA, les cytokines, les chimiokines, G-CSF, GM-CSF, les interleukines, les interférons, PDGF, TNF, RANTES, MIP1α et les facteurs de transcription.

16. Procédé selon la revendication 12 où la séquence d'acide nucléique cible code un ARN fonctionnel.

17. Acide nucléique modifié avec une région capable de transcription, qui peut être exprimé dans un système d'expression de mammifère et qui est dérivé d'une séquence de type sauvage, où la région capable de transcription est modifiée de telle manière qu'elle est optimisée quant aux codons à l'égard du système d'expression de mammifère utilisé, où un choix des codons tel qu'il est le plus fréquemment utilisé ou le second plus fréquemment utilisé dans des cellules de mammifère est utilisé, et que le nombre de dinucléotides CpG est augmenté par rapport à la séquence dérivée de la séquence de type sauvage, optimisée quant aux codons au moyen de la dégénérescence du code génétique,
où le nombre des dinucléotides CpG est augmenté par rapport à la séquence de type sauvage d'au moins cinq fois, de préférence de dix fois ou plus.

18. Acide nucléique selon la revendication 17, où l'acide nucléique n'est pas associé à un îlot de CpG.

19. Vecteur comprenant un acide nucléique selon l'une des revendications 17 à 18 en liaison fonctionnelle avec une séquence de contrôle de la transcription appropriée.

20. Vecteur selon la revendication 19 où la séquence de contrôle de la transcription comprend un promoteur.

21. Vecteur selon la revendication 20 où le promoteur est un promoteur actif de manière constitutive.

22. Vecteur selon la revendication 21 où le promoteur actif de manière constitutive est choisi parmi le promoteur de CMV (cytomégalovirus) et le promoteur du virus simien 40 (SV40).

23. Vecteur selon la revendication 20 où le promoteur est un promoteur inductible.

24. Vecteur selon la revendication 23 où le promoteur inductible est un promoteur dépendant de tétracycline.

25. Vecteur selon l'une des revendications 19 à 24 où le promoteur n'est pas associé à un îlot de CpG.

26. Vecteur selon l'une des revendications 19 à 25 où les séquences, ou des parties de celles-ci, différentes de l'acide nucléique selon l'une des revendications 17 à 18, présentes sur le vecteur comportent un nombre de dinucléotides CpG diminué.

27. Vecteur selon l'une des revendications 19 à 26 où les séquences, ou des parties de celles-ci, différentes de l'acide nucléique selon l'une des revendications 17 à 18 comportent un nombre de dinucléotides CpG diminué d'environ 25 %, de préférence de 50 %, de préférence encore de 75 %, de préférence encore de 100 %.

28. Vecteur selon l'une des revendications 20 à 27 ayant la séquence d'acide nucléique représentée dans SEQ ID NO. 25.

29. Cellule contenant un acide nucléique ou un vecteur selon l'une des revendications 17 à 28.

30. Médicament comprenant comme principe actif un vecteur et/ou une cellule selon l'une des revendications 19 à 29.

31. Utilisation d'un acide nucléique et/ou d'un vecteur et/ou d'une cellule selon l'une des revendications 18 à 29 pour la production de vaccins.

32. Acide nucléique ayant une séquence choisie parmi SEQ ID NO. 1, 5, 7, 9 et 26.


### SEQUENZPROTOKOLL

<110> Geneart GmbH
<120> Verfahren zur Modulation der Genexpression durch
   Änderung des CpG Gehalts
<130> 33252-DH
<140>
<141>
<160> 59
<170> PatentIn Ver. 2.1
<210> 1
   <211> 720
   <212> DNA
   <213> Künstliche Sequenz
<220>
<223> Beschreibung der künstlichen Sequenz:depletiertes
   delta green-fluorescent-protein (GFP) Gen
<220>
   <221> CDS
   <222> (1)..(720)
<400> 1
   atg gtg tcc aag ggg gag gag ctg ttc aca ggg gtg gtg ccc atc ctg    48
   Met Val Ser Lys Gly Glu Glu Leu Phe Thr Gly Val Val Pro Ile Leu
    1               5                  10                  15
   gtg gag ctg gat ggg gat gtg aat ggc cac aag ttc tct gtg tct ggg    96
   Val Glu Leu Asp Gly Asp Val Asn Gly His Lys Phe Ser Val Ser Gly
               20                  25                  30
   gag ggg gag ggg gat gcc acc tat ggc aag ctc acc ctg aag ttc atc   144
   Glu Gly Glu Gly Asp Ala Thr Tyr Gly Lys Leu Thr Leu Lys Phe Ile
           35                  40                  45
   tgc acc aca ggc aag ctg cca gtg ccc tgg ccc acc ctg gtg acc acc   192
   Cys Thr Thr Gly Lys Leu Pro Val Pro Trp Pro Thr Leu Val Thr Thr
       50                  55                  60
   ttc acc tat ggg gtg cag tgc ttc agc aga tac cca gac cac atg aag   240
   Phe Thr Tyr Gly Val Gln Cys Phe Ser Arg Tyr Pro Asp His Met Lys
   65                  70                  75                  80
   cag cat gac ttc ttc aag tct gcc atg cct gag ggc tat gtg cag gag   288
   Gln His Asp Phe Phe Lys Ser Ala Met Pro Glu Gly Tyr Val Gln Glu
                   85                  90                  95
   agg acc atc ttc ttc aag gat gat ggc aac tac aag acc agg gct gag   336
   Arg Thr Ile Phe Phe Lys Asp Asp Gly Asn Tyr Lys Thr Arg Ala Glu
               100                 105                 110
   gtg aag ttt gag ggg gat acc ctg gtg aac agg att gag ctg aag ggc   384
   Val Lys Phe Glu Gly Asp Thr Leu Val Asn Arg Ile Glu Leu Lys Gly
           115                 120                 125
   att gac ttt aag gag gat ggc aat atc ctg ggc cac aag ctg gag tac   432
   Ile Asp Phe Lys Glu Asp Gly Asn Ile Leu Gly His Lys Leu Glu Tyr
       130                 135                 140
   aac tac aac agc cac aat gtg tac atc atg gca gac aag cag aag aat   480
   Asn Tyr Asn Ser His Asn Val Tyr Ile Met Ala Asp Lys Gln Lys Asn
   145                 150                 155                 160
   ggc atc aag gtg aac ttc aag atc agg cac aac att gag gat ggc tct   528
   Gly Ile Lys Val Asn Phe Lys Ile Arg His Asn Ile Glu Asp Gly Ser
                   165                 170                 175
   gtg cag ctg gca gac cac tac cag cag aac acc ccc att gga gat ggc   576
   Val Gln Leu Ala Asp His Tyr Gln Gln Asn Thr Pro Ile Gly Asp Gly
               180                 185                 190
   cct gtc ctg ctg cca gac aac cac tac ctg agc acc cag tct gcc ctg   624
   Pro Val Leu Leu Pro Asp Asn His Tyr Leu Ser Thr Gln Ser Ala Leu
           195                 200                 205
   agc aag gac ccc aat gag aag agg gac cac atg gtg ctg ctg gag ttt   672
   Ser Lys Asp Pro Asn Glu Lys Arg Asp His Met Val Leu Leu Glu Phe
       210                 215                 220
   gtg aca gct gct ggc atc acc ctg ggc atg gat gag ctg tac aag tga   720
   Val Thr Ala Ala Gly Ile Thr Leu Gly Met Asp Glu Leu Tyr Lys
   225                 230                 235                 240
<210> 2
   <211> 239
   <212> PRT
   <213> Künstliche Sequenz
   <223> Beschreibung der künstlichen Sequenz:depletiertes
   delta green-fluorescent-protein (GFP) Gen
<400> 2
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 3
   <211> 720
   <212> DNA
   <213> Künstliche Sequenz
<220>
   <223> Beschreibung der künstlichen Sequenz:humanes
   green-fluorescent-protein (huGFP) Gen
<220>
   <221> CDS
   <222> (1)..(720)
<400> 3
   atg gtg agc aag ggc gag gag ctg ttc acc ggg gtg gtg ccc atc ctg    48
   Met Val Ser Lys Gly Glu Glu Leu Phe Thr Gly Val Val Pro Ile Leu
    1               5                  10                  15
   gtc gag ctg gac ggc gac gta aac ggc cac aag ttc agc gtg tcc ggc    96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc   144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc   192
   Cys Thr Thr Gly Lys Leu Pro Val Pro Trp Pro Thr Leu Val Thr Thr
       50                  55                  60
   ttc acc tac ggc gtg cag tgc ttc agc cgc tac ccc gac cac atg aag   240
   Phe Thr Tyr Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag   288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag   336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc   384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc     528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg     624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag taa     720
   Val Thr Ala Ala Gly Ile Thr Leu Gly Met Asp Glu Leu Tyr Lys
   225                 230                 235                 240
<210> 4
   <211> 239
   <212> PRT
   <213> Künstliche Sequenz
   <223> Beschreibung der künstlichen Sequenz:humanes
   green-fluorescent-protein (huGFP) Gen
<400> 4
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 5
   <211> 279
   <212> DNA
   <213> Künstliche Sequenz
<220>
   <223> Beschreibung der künstlichen Sequenz:murines
   MIP1alpha-OCpG (murines MIPlalpha-Gen mit 0
   CpG-Dinukleotiden)
<220>
   <221> CDS
   <222> (1)..(279)
<400> 5
   atg aag gtg agc aca aca gct ctg gct gtg ctg ctg tgt acc atg acc    48
   Met Lys Val Ser Thr Thr Ala Leu Ala Val Leu Leu Cys Thr Met Thr
    1               5                  10                  15
   ctg tgc aac cag gtg ttc tct gcc cct tat gga gca gat acc cct aca    96
   Leu Cys Asn Gln Val Phe Ser Ala Pro Tyr Gly Ala Asp Thr Pro Thr
               20                  25                  30
   gcc tgc tgt ttc agc tac agc agg aag atc ccc agg cag ttc att gtg    144
   Ala Cys Cys Phe Ser Tyr Ser Arg Lys Ile Pro Arg Gln Phe Ile Val
           35                  40                  45
   gac tac ttt gag acc agc agc ctg tgt tct cag cct ggg gtg atc ttt    192
   Asp Tyr Phe Glu Thr Ser Ser Leu Cys Ser Gln Pro Gly Val Ile Phe
       50                  55                  60
   ctg acc aag agg aac agg cag atc tgt gca gac agc aag gag aca tgg    240
   Leu Thr Lys Arg Asn Arg Gln Ile Cys Ala Asp Ser Lys Glu Thr Trp
   65                  70                  75                  80
   gtg cag gag tac atc aca gac ctg gag ctg aat gcc tag                279
   Val Gln Glu Tyr Ile Thr Asp Leu Glu Leu Asn Ala
                   85                  90
<210> 6
   <211> 92
   <212> PRT
   <213> Künstliche Sequenz
   <223> Beschreibung der künstlichen Sequenz:murines
   MIP1alpha-OCpG (murines MIP1alpha-Gen mit 0
   CpG-Dinukleotiden)
<400> 6
   Met Lys Val Ser Thr Thr Ala Leu Ala Val Leu Leu Cys Thr Met Thr
   1               5                   10                  15
   Leu Cys Asn Gln Val Phe Ser Ala Pro Tyr Gly Ala Asp Thr Pro Thr
               20                  25                  30
   Ala Cys Cys Phe Ser Tyr Ser Arg Lys Ile Pro Arg Gln Phe Ile Val
           35                  40                  45
   Asp Tyr Phe Glu Thr Ser Ser Leu Cys Ser Gln Pro Gly Val Ile Phe
       50                  55                  60
   Leu Thr Lys Arg Asn Arg Gln Ile Cys Ala Asp Ser Lys Glu Thr Trp
   65                  70                  75                  80
   Val Gln Glu Tyr Ile Thr Asp Leu Glu Leu Asn Ala
                   85                  90
<210> 7
   <211> 279
   <212> DNA
   <213> Künstliche Sequenz
<220>
   <223> Beschreibung der künstlichen Sequenz: murines
   MIP1alpha-2CpG (murines MIP1alpha Gen mit 2
   CpG-Dinukleotiden)
<220>
   <221> CDS
   <222> (1)..(279)
<400> 7
   atg aag gtg agc aca aca gct ctg gcc gtg ctg ctg tgt acc atg acc      48
   Met Lys Val Ser Thr Thr Ala Leu Ala Val Leu Leu Cys Thr Met Thr
    1               5                  10                  15
   ctg tgc aac cag gtg ttc tct gcc cct tat gga gca gat acc cct aca      96
   Leu Cys Asn Gln Val Phe Ser Ala Pro Tyr Gly Ala Asp Thr Pro Thr
               20                  25                  30
   gcc tgc tgt ttc agc tac agc agg aag atc ccc agg cag ttc atc gtg     144
   Ala Cys Cys Phe Ser Tyr Ser Arg Lys Ile Pro Arg Gln Phe Ile Val
           35                  40                  45
   gac tac ttt gag acc agc agc ctg tgt tct cag cct ggg gtg atc ttt     192
   Asp Tyr Phe Glu Thr Ser Ser Leu Cys Ser Gln Pro Gly Val Ile Phe
       50                  55                  60
   ctg acc aag agg aac agg cag atc tgt gca gac agc aag gag aca tgg     240
   Leu Thr Lys Arg Asn Arg Gln Ile Cys Ala Asp Ser Lys Glu Thr Trp
   65                  70                  75                  80
   gtg cag gag tac atc aca gac ctg gag ctg aat gcc tag                 279
   Val Gln Glu Tyr Ile Thr Asp Leu Glu Leu Asn Ala
                   85                  90
<210> 8
   <211> 92
   <212> PRT
   <213> Künstliche Sequenz
   <223> Beschreibung der künstlichen Sequenz: murines
   MIPlalpha-2CpG (murines MIP1alpha Gen mit 2
   CpG-Dinukleotiden)
<400> 8
   Met Lys Val Ser Thr Thr Ala Leu Ala Val Leu Leu Cys Thr Met Thr
   1               5                   10                  15
   Leu Cys Asn Gln Val Phe Ser Ala Pro Tyr Gly Ala Asp Thr Pro Thr
               20                  25                  30
   Ala Cys Cys Phe Ser Tyr Ser Arg Lys Ile Pro Arg Gln Phe Ile Val
           35                  40                  45
   Asp Tyr Phe Glu Thr Ser Ser Leu Cys Ser Gln Pro Gly Val Ile Phe
       50                  55                  60
   Leu Thr Lys Arg Asn Arg Gln Ile Cys Ala Asp Ser Lys Glu Thr Trp
   65                  70                  75                  80
   Val Gln Glu Tyr Ile Thr Asp Leu Glu Leu Asn Ala
                   85                  90
<210> 9
   <211> 279
   <212> DNA
   <213> Künstliche Sequenz
<220>
   <223> Beschreibung der künstlichen Sequenz:murines
   MIPalpha-4CpG (murines MIPalpha Gen mit 4
   CpG-Dinukleotiden)
<220>
   <221> CDS
   <222> (1)..(279)
<400> 9
   atg aag gtg agc aca aca gct ctg gcc gtg ctg ctg tgt acc atg acc      48
   Met Lys Val Ser Thr Thr Ala Leu Ala Val Leu Leu Cys Thr Met Thr
   1               5                   10                  15
   ctg tgc aac cag gtg ttc tct gcc cct tac gga gca gat acc cct aca      96
   Leu Cys Asn Gln Val Phe Ser Ala Pro Tyr Gly Ala Asp Thr Pro Thr
               20                  25                  30
   gcc tgc tgt ttc agc tac agc agg aag atc ccc agg cag ttc atc gtg     144
   Ala Cys Cys Phe Ser Tyr Ser Arg Lys Ile Pro Arg Gln Phe Ile Val
           35                  40                  45
   gac tac ttt gag acc agc agc ctg tgt tct cag cct ggg gtg atc ttt 192
   Asp Tyr Phe Glu Thr Ser Ser Leu Cys Ser Gln Pro Gly Val Ile Phe
   50 55 60
   ctg acc aag agg aac cgc cag atc tgt gca gac agc aag gag aca tgg 240
   Leu Thr Lys Arg Asn Arg Gln Ile Cys Ala Asp Ser Lys Glu Thr Trp
   65 70 75 80
   gtg cag gag tac atc aca gac ctg gag ctg aat gcc tag 279
   Val Gln Glu Tyr Ile Thr Asp Leu Glu Leu Asn Ala
   85 90
<210> 10
   <211> 92
   <212> PRT
   <213> Künstliche Sequenz
   <223> Beschreibung der künstlichen Sequenz:murines
   MIPalpha-4CpG (murines MIPalpha Gen mit 4
   CpG-Dinukleotiden)
<400> 10
   Met Lys Val Ser Thr Thr Ala Leu Ala Val Leu Leu Cys Thr Met Thr
   1 5 10 15
   Leu Cys Asn Gln Val Phe Ser Ala Pro Tyr Gly Ala Asp Thr Pro Thr
   20 25 30
   Ala Cys Cys Phe Ser Tyr Ser Arg Lys Ile Pro Arg Gln Phe Ile Val
   35 40 45
   Asp Tyr Phe Glu Thr Ser Ser Leu Cys Ser Gln Pro Gly Val Ile Phe
   50 55 60
   Leu Thr Lys Arg Asn Arg Gln Ile Cys Ala Asp Ser Lys Glu Thr Trp
   65 70 75 80
   Val Gln Glu Tyr Ile Thr Asp Leu Glu Leu Asn Ala
   85 90
<210> 11
   <211> 279
   <212> DNA
   <213> Künstliche Sequenz
<220>
   <223> Beschreibung der künstlichen Sequenz:murines
   MIPalpha-13CpG (murines MIPalpha Gen mit 13
   CpG-Dinukleotiden)
<220>
   <221> CDS
   <222> (1)..(279)
<400> 11
   atg aag gtg agc acc aca gct ctg gct gtg ctg ctg tgc acc atg acc     48
   Met Lys Val Ser Thr Thr Ala Leu Ala Val Leu Leu Cys Thr Met Thr
   1               5                   10                  15
   ctg tgc aac cag gtg ttc agc gct cct tac ggc gcc gat acc cct aca     96
   Leu Cys Asn Gln Val Phe Ser Ala Pro Tyr Gly Ala Asp Thr Pro Thr
               20                  25                  30
   gcc tgc tgc ttc agc tac agc agg aag atc ccc agg cag ttc atc gtg    144
   Ala Cys Cys Phe Ser Tyr Ser Arg Lys Ile Pro Arg Gln Phe Ile Val
           35                  40                  45
   gac tac ttc gag acc agc agc ctg tgt tct cag ccc ggc gtg atc ttc    192
   Asp Tyr Phe Glu Thr Ser Ser Leu Cys Ser Gln Pro Gly Val Ile Phe
       50                  55                  60
   ctg acc aag cgg aac aga cag atc tgc gcc gac agc aag gag aca tgg    240
   Leu Thr Lys Arg Asn Arg Gln Ile Cys Ala Asp Ser Lys Glu Thr Trp
   65                  70                  75                  80
   gtg cag gag tac atc acc gac ctg gag ctg aac gcc tag                279
   Val Gln Glu Tyr Ile Thr Asp Leu Glu Leu Asn Ala
                   85                  90
<210> 12
   <211> 92
   <212> PRT
   <213> Künstliche Sequenz
   <223> Beschreibung der künstlichen Sequenz:murines
   MIPalpha-13CpG (murines MIPalpha Gen mit 13
   CpG-Dinukleotiden)
<400> 12
   Met Lys Val Ser Thr Thr Ala Leu Ala Val Leu Leu Cys Thr Met Thr
   1               5                   10                  15
   Leu Cys Asn Gln Val Phe Ser Ala Pro Tyr Gly Ala Asp Thr Pro Thr
               20                  25                  30
   Ala Cys Cys Phe Ser Tyr Ser Arg Lys Ile Pro Arg Gln Phe Ile Val
           35                  40                  45
   Asp Tyr Phe Glu Thr Ser Ser Leu Cys Ser Gln Pro Gly Val Ile Phe
       50                  55                  60
   Leu Thr Lys Arg Asn Arg Gln Ile Cys Ala Asp Ser Lys Glu Thr Trp
   65                  70                  75                  80
   Val Gln Glu Tyr Ile Thr Asp Leu Glu Leu Asn Ala
                   85                  90
<210> 13
   <211> 279
   <212> DNA
   <213> Künstliche Sequenz
<220>
   <223> Beschreibung der künstlichen Sequenz:murines
   MIPalpha-42CpG (murines MIPalpha Gen mit 42
   CpG-Dinukleotiden)
<220>
   <221> CDS
   <222> (1)..(279)
<400> 13
   atg aag gtg tcg acg acc gcg ctc gcc gtg ctg ctg tgc acg atg acg    48
   Met Lys Val Ser Thr Thr Ala Leu Ala Val Leu Leu Cys Thr Met Thr
   1               5                   10                  15
   ctg tgc aac cag gtg ttc agc gcc ccg tac ggc gcc gac acg ccg acc    96
   Leu Cys Asn Gln Val Phe Ser Ala Pro Tyr Gly Ala Asp Thr Pro Thr
               20                  25                  30
   gcg tgc tgc ttc tcg tac tcg cgg aag atc ccg cgg cag ttc atc gtc    144
   Ala Cys Cys Phe Ser Tyr Ser Arg Lys Ile Pro Arg Gln Phe Ile Val
           35                  40                  45
   gac tac ttc gaa acg tcg tcg ctg tgc tcg cag ccc ggc gtg atc ttc    192
   Asp Tyr Phe Glu Thr Ser Ser Leu Cys Ser Gln Pro Gly Val Ile Phe
       50                  55                  60
   ctc acg aag cgg aac cgg cag atc tgc gcc gac tcg aag gaa acg tgg    240
   Leu Thr Lys Arg Asn Arg Gln Ile Cys Ala Asp Ser Lys Glu Thr Trp
   65                  70                  75                  80
   gtg cag gag tac atc acc gac ctc gaa ctg aac gcg tag                279
   Val Gln Glu Tyr Ile Thr Asp Leu Glu Leu Asn Ala
                   85                  90
<210> 14
   <211> 92
   <212> PRT
   <213> Künstliche Sequenz
   <223> Beschreibung der künstlichen Sequenz:murines
   MIPalpha-42CpG (murines MIPalpha Gen mit 42
   CpG-Dinukleotiden)
<400> 14
   Met Lys Val Ser Thr Thr Ala Leu Ala Val Leu Leu Cys Thr Met Thr
   1               5                   10                  15
   Leu Cys Asn Gln Val Phe Ser Ala Pro Tyr Gly Ala Asp Thr Pro Thr
               20                  25                  30
   Ala Cys Cys Phe Ser Tyr Ser Arg Lys Ile Pro Arg Gln Phe Ile Val
           35                  40                  45
   Asp Tyr Phe Glu Thr Ser Ser Leu Cys Ser Gln Pro Gly Val Ile Phe
       50                  55                  60
   Leu Thr Lys Arg Asn Arg Gln Ile Cys Ala Asp Ser Lys Glu Thr Trp
   65                  70                  75                  80
   Val Gln Glu Tyr Ile Thr Asp Leu Glu Leu Asn Ala
                   85                  90
<210> 15
   <211> 279
   <212> DNA
   <213> murin
<220>
   <223> murines MIPlalpha Wildtyp mit 7 CpG-Dinukleotiden
<220>
   <221> CDS
   <222> (1)..(279)
<400> 15
   atg aag gtc tcc acc act gcc ctt gct gtt ctt ctc tgt acc atg aca      48
   Met Lys Val Ser Thr Thr Ala Leu Ala Val Leu Leu Cys Thr Met Thr
   1               5                   10                  15
   ctc tgc aac caa gtc ttc tca gcg cca tat gga gct gac acc ccg act      96
   Leu Cys Asn Gln Val Phe Ser Ala Pro Tyr Gly Ala Asp Thr Pro Thr
               20                  25                  30
   gcc tgc tgc ttc tcc tac agc cgg aag att cca cgc caa ttc atc gtt      144
   Ala Cys Cys Phe Ser Tyr Ser Arg Lys Ile Pro Arg Gln Phe Ile Val
           35                  40                  45
   gac tat ttt gaa acc agc agc ctt tgc tcc cag cca ggt gtc att ttc      192
   Asp Tyr Phe Glu Thr Ser Ser Leu Cys Ser Gln Pro Gly Val Ile Phe
       50                  55                  60
   ctg act aag aga aac cgg cag atc tgc gct gac tcc aaa gag acc tgg      240
   Leu Thr Lys Arg Asn Arg Gln Ile Cys Ala Asp Ser Lys Glu Thr Trp
   65                  70                  75                  80
   gtc caa gaa tac atc act gac ctg gaa ctg aat gcc tag                  279
   Val Gln Glu Tyr Ile Thr Asp Leu Glu Leu Asn Ala
                   85                  90
<210> 16
   <211> 92
   <212> PRT
   <213> murin
   <223> murines MIP1alpha Wildtyp mit 7 CpG-Dinukleotiden
<400> 16
   Met Lys Val Ser Thr Thr Ala Leu Ala Val Leu Leu Cys Thr Met Thr
   1               5                   10                  15
   Leu Cys Asn Gln Val Phe Ser Ala Pro Tyr Gly Ala Asp Thr Pro Thr
               20                  25                  30
   Ala Cys Cys Phe Ser Tyr Ser Arg Lys Ile Pro Arg Gln Phe Ile Val
           35                  40                  45
   Asp Tyr Phe Glu Thr Ser Ser Leu Cys Ser Gln Pro Gly Val Ile Phe
       50                  55                  60
   Leu Thr Lys Arg Asn Arg Gln Ile Cys Ala Asp Ser Lys Glu Thr Trp
   65                  70                  75                  80
   Val Gln Glu Tyr Ile Thr Asp Leu Glu Leu Asn Ala
                   85                  90
<210> 17
   <211> 282
   <212> DNA
   <213> Künstliche Sequenz
<220>
   <223> Beschreibung der künstlichen Sequenz:humanes
   MIP1alpha-43CpG (humanes MIP1alpha-Gen mit 43 CpG)
<220>
   <221> CDS
   <222> (1)..(282)
<400> 17
   atg caa gtg tcg acc gcc gct ctc gcc gtg ctg ctg tgc acg atg gcg     48
   Met Gln Val Ser Thr Ala Ala Leu Ala Val Leu Leu Cys Thr Met Ala
    1               5                  10                  15
   ctg tgc aac caa gtg ctg agc gcg cct ctc gcc gcc gac acg ccg acc     96
   Leu Cys Asn Gln Val Leu Ser Ala Pro Leu Ala Ala Asp Thr Pro Thr
               20                  25                  30
   gcg tgc tgc ttc tcg tac acg tcg cgg cag atc ccg cag aac ttc atc    144
   Ala Cys Cys Phe Ser Tyr Thr Ser Arg Gln Ile Pro Gln Asn Phe Ile
           35                  40                  45
   gcc gac tac ttc gag acg tcg tcg cag tgc tcg aag ccg agc gtg atc    192
   Ala Asp Tyr Phe Glu Thr Ser Ser Gln Cys Ser Lys Pro Ser Val Ile
       50                  55                  60
   ttc ctg acg aag cgc gga cgg caa gtg tgc gcc gac ccg agc gag gag    240
   Phe Leu Thr Lys Arg Gly Arg Gln Val Cys Ala Asp Pro Ser Glu Glu
   65                  70                  75                  80
   tgg gtg cag aag tac gtg agc gac ctc gaa ctg agc gcg tag            282
   Trp Val Gln Lys Tyr Val Ser Asp Leu Glu Leu Ser Ala
                   85                  90
<210> 18
   <211> 93
   <212> PRT
   <213> Künstliche Sequenz
   <223> Beschreibung der künstlichen Sequenz:humanes
   MIPlalpha-43CpG (humanes MIP1alpha-Gen mit 43 CpG)
<400> 18
   Met Gln Val Ser Thr Ala Ala Leu Ala Val Leu Leu Cys Thr Met Ala
   1               5                   10                  15
   Leu Cys Asn Gln Val Leu Ser Ala Pro Leu Ala Ala Asp Thr Pro Thr
               20                  25                  30
   Ala Cys Cys Phe Ser Tyr Thr Ser Arg Gln Ile Pro Gln Asn Phe Ile
           35                  40                  45
   Ala Asp Tyr Phe Glu Thr Ser Ser Gln Cys Ser Lys Pro Ser Val Ile
       50                  55                  60
   Phe Leu Thr Lys Arg Gly Arg Gln Val Cys Ala Asp Pro Ser Glu Glu
   65                  70                  75                  80
   Trp Val Gln Lys Tyr Val Ser Asp Leu Glu Leu Ser Ala
                   85                  90
<210> 19
   <211> 435
   <212> DNA
   <213> Künstliche Sequenz
<220>
   <223> Beschreibung der künstlichen Sequenz:synthetisches
   Gen
<220>
   <223> humanes GM-CSF-63CpG
<220>
   <221> CDS
   <222> (1)..(435)
<400> 19
<210> 20
   <211> 144
   <212> PRT
   <213> Künstliche Sequenz
   <223> humanes GM-CSF-63CpG
<400> 20
   Met Trp Leu Gln Ser Leu Leu Leu Leu Gly Thr Val Ala Cys Ser Ile
   1 5 10 15
   Ser Ala Pro Ala Arg Ser Pro Ser Pro Ser Thr Gln Pro Trp Glu His
   20 25 30
   Val Asn Ala Ile Gln Glu Ala Arg Arg Leu Leu Asn Leu Ser Arg Asp
   35 40 45
   Thr Ala Ala Glu Met Asn Glu Thr Val Glu Val Ile Ser Glu Met Phe
   50 55 60
   Asp Leu Gln Glu Pro Thr Cys Leu Gln Thr Arg Leu Glu Leu Tyr Lys
   65 70 75 80
   Gln Gly Leu Arg Gly Ser Leu Thr Lys Leu Lys Gly Pro Leu Thr Met
   85 90 95
   Met Ala Ser His Tyr Lys Gln His Cys Pro Pro Thr Pro Glu Thr Ser
   100 105 110
   Cys Ala Thr Gln Ile Ile Thr Phe Glu Ser Phe Lys Glu Asn Leu Lys
   115 120 125
   Asp Phe Leu Leu Val Ile Pro Phe Asp Cys Trp Glu Pro Val Gln Glu
   130 135 140
<210> 21
   <211> 282
   <212> DNA
   <213> human
<220>
   <223> humanes MIP1alpha Gen Wildtyp (8CpG)
<220>
   <221> CDS
   <222> (1)..(282)
<400> 21
   atg cag gtc tcc act gct gcc ctt gcc gtc ctc ctc tgc acc atg gct      48
   Met Gln Val Ser Thr Ala Ala Leu Ala Val Leu Leu Cys Thr Met Ala
    1               5                  10                  15
   ctc tgc aac cag gtc ctc tct gca cca ctt gct gct gac acg ccg acc      96
   Leu Cys Asn Gln Val Leu Ser Ala Pro Leu Ala Ala Asp Thr Pro Thr
               20                  25                  30
   gcc tgc tgc ttc agc tac acc tcc cga cag att cca cag aat ttc ata     144
   Ala Cys Cys Phe Ser Tyr Thr Ser Arg Gln Ile Pro Gln Asn Phe Ile
           35                  40                  45
   gct gac tac ttt gag acg agc agc cag tgc tcc aag ccc agt gtc atc     192
   Ala Asp Tyr Phe Glu Thr Ser Ser Gln Cys Ser Lys Pro Ser Val Ile
       50                  55                  60
   ttc cta acc aag aga ggc cgg cag gtc tgt gct gac ccc agt gag gag     240
   Phe Leu Thr Lys Arg Gly Arg Gln Val Cys Ala Asp Pro Ser Glu Glu
   65                  70                  75                  80
   tgg gtc cag aaa tac gtc agt gac ctg gag ctg agt gcc tag             282
   Trp Val Gln Lys Tyr Val Ser Asp Leu Glu Leu Ser Ala
                       85                  90
<210> 22
   <211> 93
   <212> PRT
   <213> human
   <223> humanes MIP1alpha Gen Wildtyp (8CpG)
<400> 22
   Met Gln Val Ser Thr Ala Ala Leu Ala Val Leu Leu Cys Thr Met Ala
    1               5                  10                  15
   Leu Cys Asn Gln Val Leu Ser Ala Pro Leu Ala Ala Asp Thr Pro Thr
               20                  25                  30
   Ala Cys Cys Phe Ser Tyr Thr Ser Arg Gln Ile Pro Gln Asn Phe Ile
           35                  40                  45
   Ala Asp Tyr Phe Glu Thr Ser Ser Gln Cys Ser Lys Pro Ser Val Ile
       50                  55                  60
   Phe Leu Thr Lys Arg Gly Arg Gln Val Cys Ala Asp Pro Ser Glu Glu
   65                  70                  75                  80
   Trp Val Gln Lys Tyr Val Ser Asp Leu Glu Leu Ser Ala
                   85                  90
<210> 23
   <211> 435
   <212> DNA
   <213> human
<220>
   <223> humanes GM-CSF Gen Wildtyp (10 CpG)
<220>
   <221> CDS
   <222> (1)..(935)
<400> 23
<210> 24
   <211> 144
   <212> PRT
   <213> human
   <223> humanes GM-CSF Gen Wildtyp (10 CpG)
<400> 24
   Met Trp Leu Gln Ser Leu Leu Leu Leu Gly Thr Val Ala Cys Ser Ile
   1 5 10 15
   Ser Ala Pro Ala Arg Ser Pro Ser Pro Ser Thr Gln Pro Trp Glu His
   20 25 30
   Val Asn Ala Ile Gln Glu Ala Arg Arg Leu Leu Asn Leu Ser Arg Asp
   35 40 45
   Thr Ala Ala Glu Met Asn Glu Thr Val Glu Val Ile Ser Glu Met Phe
   50 55 60
   Asp Leu Gln Glu Pro Thr Cys Leu Gln Thr Arg Leu Glu Leu Tyr Lys
   65 70 75 80
   Gln Gly Leu Arg Gly Ser Leu Thr Lys Leu Lys Gly Pro Leu Thr Met
   85 90 95
   Met Ala Ser His Tyr Lys Gln His Cys Pro Pro Thr Pro Glu Thr Ser
   100 105 110
   Cys Ala Thr Gln Ile Ile Thr Phe Glu Ser Phe Lys Glu Asn Leu Lys
   115 120 125
   Asp Phe Leu Leu Val Ile Pro Phe Asp Cys Trp Glu Pro Val Gln Glu
   130 135 140
<210> 25
   <211> 3034
   <212> DNA
   <213> Künstliche Sequenz
<220>
   <223> Beschreibung der künstlichen Sequenz:p smallsyn
   Vektor
<400> 25
   atcgatgttg acattgatta ttgactagtt attaatagta atcaattacg gggtcattag 60
   ttcatagccc atatatggag ttccgcgtta cataacttac ggtaaatggc ccgcctggct 120
   gaccgcccaa cgacccccgc ccattgacgt caataatgac gtatgttccc atagtaacgc 180
   caatagggac tttccattga cgtcaatggg tggagtattt acggtaaact gcccacttgg 240
   cagtacatca agtgtatcat atgccaagta cgccccctat tgacgtcaat gacggtaaat 300
   ggcccgcctg gcattatgcc cagtacatga ccttatggga ctttcctact tggcagtaca 360
   tctacgtatt agtcatcgct attaccatgg tgatgcggtt ttggcagtac atcaatgggc 420
   gtggatagcg gtttgactca cggggatttc caagtctcca ccccattgac gtcaatggga 480
   gtttgttttg gcaccaaaat caacgggact ttccaaaatg tcgtaacaac tccgccccat 540
   tgacgcaaat gggcggtagg cgtgtacggt gggaggtcta tataagcaga gctctctggc 600
   taactagaga acccactgct tactggctta tctaaattaa tacgactcac tatagggaga 660
   cccaagctgt taagcttggt agatatcagg gatccactca gctgatcagc ctccagttta 720
   aacctgtgcc ttctagttgc cagccatctg ttgtttgccc ctcccccgtg ccttccttga 780
   ccctggaagg tgccactccc actgtccttt cctaataaaa tgaggaaatt gcatcgcatt 840
   gtctgagtag gtgtcattct attctggggg gtggggtggg gcaggacagc aagggggagg 900
   attgggaaga caatagcagg catgctgggg atgcggtggg ctctatggcc cgggtagtga 960
   attcatgtga gcaaaaggcc agcaaaaggc caggaaccgt aaaaaggccg cgttgctggc 1020
   gtttttccat aggctccgcc cccctgacga gcatcacaaa aatcgacgct caagtcagag 1080
   gtggcgaaac ccgacaggac tataaagata ccaggcgttt ccccctggaa gctccctcgt 1140
   gcgctctcct gttccgaccc tgccgcttac cggatacctg tccgcctttc tcccttcggg 1200
   aagcgtggcg ctttctcata gctcacgctg taggtatctc agttcggtgt aggtcgttcg 1260
   ctccaagctg ggctgtgtgc acgaaccccc cgttcagccc gaccgctgcg ccttatccgg 1320
   taactatcgt cttgagtcca acccggtaag acacgactta tcgccactgg cagcagccac 1380
   tggtaacagg attagcagag cgaggtatgt aggcggtgct acagagttct tgaagtggtg 1440
   gcctaactac ggctacacta gaaggacagt atttggtatc tgcgctctgc tgaagccagt 1500
   taccttcgga aaaagagttg gtagctcttg atccggcaaa caaaccaccg ctggtagcgg 1560
   tggttttttt gtttgcaagc agcagattac gcgcagaaaa aaaggatctc aagaagatcc 1620
   tttgatcttt tctacgggag atctgtctga ctctcagtgg aaccaaaact catgttaagg 1680
   gattttggtc atgagattat caaaaaggat cttcacctag atccttttaa attaaaaatg 1740
   aagttttaaa tcaatctaaa gtatatatga gtaaacttgg tctgacaggt taacttacca 1800
   atgcttaatc aatgaggcac caatctctgc aatctgccta tttctctcat ccatggttgc 1860
   ctgactgcct gtggtgtaga taactacaat cctggagggc ttaccatctg gccccagtgc 1920
   tgcaatgata cctctagacc ctctctcacc tgctccagat ttatctgcaa tgaaccagcc 1980
   agctggaagg gcagacctca gaagtggtcc tgcaacttta tctgcctcca tccagtctat 2040
   taattgttgt ctggaagcta gagtaagcag ttcaccagtt aatagtttcc tcaaggttgt 2100
   tgccattgct acaggcatgg tggtgtccct ctcatcattt ggtatggctt cattcagctc 2160
   tggttcccat ctatcaagcc tagttacatg atcacccatg ttgtgcaaaa aagcagtcaa 2220
   ctcctttggt cctccaatgg ttgtcaaaag taagttggca gcagtgttat cactcatggt 2280
   tatggcagca ctgcataatt ctcttactgt catgccatct gtaagatgct tttctgtgac 2340
   tggactgtac tcaaccaagt cattctgaga atagtgtatt cttctaccca gttgctcttg 2400
   cccagcatca attctggata atactgcacc acatagcaga actttaaagg tgctcatcat 2460
   tggaaatctt tcttctggtc taaaactctc aaggatctta ccagagttga gatccagttc 2520
   aatgtaaccc actcttgcac ccaactgatc ttcagcatct tttactttca ccagggtttc 2580
   tgggtgagca aaaacaggaa ggcaaaaggc agcaaaaaag ggaataaggg caactctgaa 2640
   atgttgaata ctcatagtac tactcttcct ttttcaatat tattgaagca tttatcaggg 2700
   ttattgtctc atgagcggat acatatttga atgtatttag aaaaataaac aaataggggt 2760
   atgcattcag ctcacatttc cctgaaaagt gccacctgaa attgactgat agggagttct 2820
   cccaatcccc tatggtgcac tctcagtaca atctgctctg atgcctcata gttaagccag 2880
   tatctgctcc ctgcttgtgt gttggaggtc actgagtagt gggctagcaa aatttaagct 2940
   acaacaaggc aaggcttgac ctacaattgc atgaagaatc tgcttagggt taggcctttt 3000
   gcactgcttg gagatgtact ggccagatat acta                            3034
<210> 26
   <211> 669
   <212> DNA
   <213> Künstliche Sequenz
<220>
   <223> Beschreibung der künstlichen Sequenz:p24 delta CpG
   Gen (HIV-1 Plasmid p24 (Kapsidprotein) Gen mit
   depletierten CpG-Dinukleotiden)
<220>
   <221> CDS
   <222> (1)..(696)
<400> 26
   atg gtg cac cag gcc atc agc ccc agg acc ctg aat gcc tgg gtg aag      48
   Met Val His Gln Ala Ile Ser Pro Arg Thr Leu Asn Ala Trp Val Lys
    1               5                  10                  15
   gtg gtg gag gag aag gcc ttc agc cct gag gtg atc ccc atg ttc tct      96
   Val Val Glu Glu Lys Ala Phe Ser Pro Glu Val Ile Pro Met Phe Ser
               20                  25                  30
   gcc ctg tct gag ggg gcc acc ccc cag gac ctg aac acc atg ctg aac     144
   Ala Leu Ser Glu Gly Ala Thr Pro Gln Asp Leu Asn Thr Met Leu Asn
           35                  40                  45
   aca gtg ggg ggc cac cag gct gcc atg cag atg ctg aag gaa acc atc     192
   Thr Val Gly Gly His Gln Ala Ala Met Gln Met Leu Lys Glu Thr Ile
       50                  55                  60
   aat gag gag gct gct gag tgg gac aga gtg cac cct gtg cat gct ggc     240
   Asn Glu Glu Ala Ala Glu Trp Asp Arg Val His Pro Val His Ala Gly
   65                  70                  75                  80
   ccc att gcc cct ggc cag atg agg gag ccc agg ggc tct gac att gct     288
   Pro Ile Ala Pro Gly Gln Met Arg Glu Pro Arg Gly Ser Asp Ile Ala
                   85                  90                  95
   ggc acc acc tcc acc ctg cag gag cag att ggc tgg atg acc aac aac     336
   Gly Thr Thr Ser Thr Leu Gln Glu Gln Ile Gly Trp Met Thr Asn Asn
               100                 105                 110
   ccc ccc atc cct gtg ggg gag atc tac aag aga tgg atc atc ctg ggc     384
   Pro Pro Ile Pro Val Gly Glu Ile Tyr Lys Arg Trp Ile Ile Leu Gly
           115                 120                 125
   ctg aac aag att gtg agg atg tac agc ccc acc tcc atc ctg gac atc     432
   Leu Asn Lys Ile Val Arg Met Tyr Ser Pro Thr Ser Ile Leu Asp Ile
       130                 135                 140
   agg cag ggc ccc aag gag ccc ttc agg gac tat gtg gac agg ttc tac     480
   Arg Gln Gly Pro Lys Glu Pro Phe Arg Asp Tyr Val Asp Arg Phe Tyr
   145                 150                 155                 160
   aag acc ctg agg gct gag cag gcc agc cag gag gtg aag aac tgg atg     528
   Lys Thr Leu Arg Ala Glu Gln Ala Ser Gln Glu Val Lys Asn Trp Met
                   165                 170                 175
   aca gag acc ctg ctg gtg cag aat gcc aac cct gac tgc aag acc atc     576
   Thr Glu Thr Leu Leu Val Gln Asn Ala Asn Pro Asp Cys Lys Thr Ile
               180                 185                 190
   ctg aag gcc ctg ggc cca gct gcc acc ctg gag gag atg atg aca gcc     624
   Leu Lys Ala Leu Gly Pro Ala Ala Thr Leu Glu Glu Met Met Thr Ala
           195                 200                 205
   tgc cag ggg gtg gga ggc cct ggc cac aag gcc agg gtg ctg taa         669
   Cys Gln Gly Val Gly Gly Pro Gly His Lys Ala Arg Val Leu
       210                 215                 220
<210> 27
   <211> 222
   <212> PRT
   <213> Künstliche Sequenz
   <223> Beschreibung der künstlichen Sequenz:p24 delta CpG
   Gen (HIV-1 Plasmid p24 (Kapsidprotein) Gen mit
   depletierten CpG-Dinukleotiden)
<400> 27
   Met Val His Gln Ala Ile Ser Pro Arg Thr Leu Asn Ala Trp Val Lys
   1               5                   10                  15
   Val Val Glu Glu Lys Ala Phe Ser Pro Glu Val Ile Pro Met Phe Ser
               20                  25                  30
   Ala Leu Ser Glu Gly Ala Thr Pro Gln Asp Leu Asn Thr Met Leu Asn
           35                  40                  45
   Thr Val Gly Gly His Gln Ala Ala Met Gln Met Leu Lys Glu Thr Ile
       50                  55                  60
   Asn Glu Glu Ala Ala Glu Trp Asp Arg Val His Pro Val His Ala Gly
   65                  70                  75                  80
   Pro Ile Ala Pro Gly Gln Met Arg Glu Pro Arg Gly Ser Asp Ile Ala
                   85                  90                  95
   Gly Thr Thr Ser Thr Leu Gln Glu Gln Ile Gly Trp Met Thr Asn Asn
               100                 105                 110
   Pro Pro Ile Pro Val Gly Glu Ile Tyr Lys Arg Trp Ile Ile Leu Gly
           115                 120                 125
   Leu Asn Lys Ile Val Arg Met Tyr Ser Pro Thr Ser Ile Leu Asp Ile
       130                 135                 140
   Arg Gln Gly Pro Lys Glu Pro Phe Arg Asp Tyr Val Asp Arg Phe Tyr
   145                 150                 155                 160
   Lys Thr Leu Arg Ala Glu Gln Ala Ser Gln Glu Val Lys Asn Trp Met
                   165                 170                 175
   Thr Glu Thr Leu Leu Val Gln Asn Ala Asn Pro Asp Cys Lys Thr Ile
               180                 185                 190
   Leu Lys Ala Leu Gly Pro Ala Ala Thr Leu Glu Glu Met Met Thr Ala
           195                 200                 205
   Cys Gln Gly Val Gly Gly Pro Gly His Lys Ala Arg Val Leu
       210                 215                 220
<210> 28
   <211> 34
   <212> DNA
   <213> Künstliche Sequenz
<220>
   <223> Beschreibung der künstlichen Sequenz:Primer
   huGFP-1
<400> 28
   caataagctt gccaccatgg tgagcaaggg cgag 34
<210> 29
   <211> 29
   <212> DNA
   <213> Künstliche Sequenz
<220>
   <223> Beschreibung der künstlichen Sequenz:Primer
   huGFP-2
<400> 29
   agtaggatcc tattacttgt acagctcgt 29
<210> 30
   <211> 20
   <212> DNA
   <213> Künstliche Sequenz
<220>
   <223> Beschreibung der künstlichen Sequenz:Primer
   RT-oligol
<400> 30
   ccctgaagtt catctgcacc 20
<210> 31
   <211> 22
   <212> DNA
   <213> Künstliche Sequenz
<220>
   <223> Beschreibung der künstlichen Sequenz:Primer oligo2
<400> 31
   gatcttgaag ttcaccttga tg 22
<210> 32
   <211> 34
   <212> DNA
   <213> Künstliche Sequenz
<220>
   <223> Beschreibung der künstlichen Sequenz:Primer
   mamip-1
<400> 32
   caggtaccaa gcttatgaag gtctccacca ctgc 34
<210> 33
   <211> 43
   <212> DNA
   <213> Künstliche Sequenz
<220>
   <223> Beschreibung der künstlichen Sequenz:Primer
   mamip-2
<400> 33
   cagagctcga gtcatgaaga ctaggcattc agttccaggt cag 43
<210> 34
   <211> 33
   <212> DNA
   <213> Künstliche Sequenz
<220>
   <223> Beschreibung der künstlichen Sequenz:Primer hugm-1
<400> 34
   caggtaccaa gcttatgtgg ctgcagagcc tgc 33
<210> 35
   <211> 43
   <212> DNA
   <213> Künstliche Sequenz
<220>
   <223> Beschreibung der künstlichen Sequenz:Primer hugm-2
<400> 35
   cagagctcga gtcatgaaga ctactcctgg actggctccc agc 43
<210> 36
   <211> 33
   <212> DNA
   <213> Künstliche Sequenz
<220>
   <223> Beschreibung der künstlichen Sequenz:Primer
   humip-1
<400> 36
   cagtaccaag cttatgcagg tctccactgc tgc 33
<210> 37
   <211> 45
   <212> DNA
   <213> Künstliche Sequenz
<220>
   <223> Beschreibung der künstlichen Sequenz:Primer
   humip-2
<400> 37
   cagagctcga gtcatgaaga ctaggcactc agctccaggt cactg 45
<210> 38
   <211> 43
   <212> DNA
   <213> Künstliche Sequenz
<220>
   <223> Beschreibung der künstlichen Sequenz:Primer p24-1
<400> 38
   actaggtacc atctaagctt atgcccatcg tgcagaacat cca 43
<210> 39
   <211> 45
   <212> DNA
   <213> Künstliche Sequenz
<220>
   <223> Beschreibung der künstlichen Sequenz:Primer p24-2
<400> 39
   tcaagagctc gactggatcc tattacagca ccctggcctt gtggc 45
<210> 40
   <211> 41
   <212> DNA
   <213> Künstliche Sequenz
<220>
   <223> Beschreibung der künstlichen Sequenz:Primer CMV-1
<400> 40
   caaaggtacc gttaatcgat gttgacattg attattgact a 41
<210> 41
   <211> 24
   <212> DNA
   <213> Künstliche Sequenz
<220>
   <223> Beschreibung der künstlichen Sequenz:Primer CMV-2
<400> 41
   gaatgagctc tgcttatata gacc 24
<210> 42
   <211> 35
   <212> DNA
   <213> Künstliche Sequenz
<220>
   <223> Beschreibung der künstlichen Sequenz:Primer ori-1
<400> 42
   gtcacccggg tagtgaattc atgtgagcaa aaggc 35
<210> 43
   <211> 35
   <212> DNA
   <213> Künstliche Sequenz
<220>
   <223> Beschreibung der künstlichen Sequenz:Primer ori-2
<400> 43
   gatcttttct acgggagatc tgtcaatcga tagct 35
<210> 44
   <211> 42
   <212> DNA
   <213> Künstliche Sequenz
<220>
   <223> Beschreibung der künstlichen Sequenz:Primer pa-1
<400> 44
   gttagagctc cagtgtttaa acctgtgcct tctagttgcc ag 42
<210> 45
   <211> 39
   <212> DNA
   <213> Künstliche Sequenz
<220>
   <223> Beschreibung der künstlichen Sequenz:Primer pa-2
<400> 45
   caaacctacc gatacccggg ccatagagcc caccgcatc 39
<210> 46
   <211> 42
   <212> DNA
   <213> Künstliche Sequenz
<220>
   <223> Beschreibung der künstlichen Sequenz:Primer
   ref-del-1
<400> 46
   tcagatgcat ccgtacgtta acatgtgagc aaaaggccag ca 42
<210> 47
   <211> 33
   <212> DNA
   <213> Künstliche Sequenz
<220>
   <223> Beschreibung der künstlichen Sequenz:Primer
   ref-del-2
<400> 47
   agtcatgcat ccatagagcc caccgcatcc cca 33
<210> 48
   <211> 33
   <212> DNA
   <213> Künstliche Sequenz
<220>
   <223> Beschreibung der künstlichen Sequenz:primer 5'-3
<220>
   <223> huil-1
<400> 48
   caggtaccaa gcttatgaga atttcgaaac cac 33
<210> 49
   <211> 45
   <212> DNA
   <213> Künstliche Sequenz
<220>
   <223> Beschreibung der künstlichen Sequenz:primer 5-3
<220>
   <223> huil-2
<400> 49
   cagagctcga gtcatgaaga ctaagaagtg ttgatgaaca tttgg 45
<210> 50
   <211> 34
   <212> DNA
   <213> Künstliche Sequenz
<220>
   <223> Beschreibung der künstlichen Sequenz:primer 5-3
<220>
   <223> magm-1
<400> 50
   caggtaccaa gcttatggcc cacgagagaa aggc 34
<210> 51
   <211> 43
   <212> DNA
   <213> Künstliche Sequenz
<220>
   <223> Beschreibung der künstlichen Sequenz:primer 5' -3
<220>
   <223> magm-2
<400> 51
   cagagctcga gtcatgaaga ctatttttgg cctggttttt tgc 43
<210> 52
   <211> 489
   <212> DNA
   <213> Künstliche Sequenz
<220>
   <223> Beschreibung der künstlichen Sequenz:synthetisches
   Gen
<220>
   <223> humanes IL-15-21CpG
<220>
   <221> CDS
   <222> (1)..(489)
<400> 52
   atg cgg atc agc aag ccc cac ctg agg agc atc agc atc cag tgc tac      48
   Met Arg Ile Ser Lys Pro His Leu Arg Ser Ile Ser Ile Gln Cys Tyr
   1               5                   10                  15
   ctg tgc ctg ctg ctg aac agc cac ttc ctg aca gag gcc ggc atc cac      96
   Leu Cys Leu Leu Leu Asn Ser His Phe Leu Thr Glu Ala Gly Ile His
               20                  25                  30
   gtg ttt atc ctg ggc tgc ttc tct gcc ggc ctg cct aag aca gag gcc      144
   Val Phe Ile Leu Gly Cys Phe Ser Ala Gly Leu Pro Lys Thr Glu Ala
           35                  40                  45
   aac tgg gtg aac gtg atc agc gac ctg aag aag atc gag gac ctg atc      192
   Asn Trp Val Asn Val Ile Ser Asp Leu Lys Lys Ile Glu Asp Leu Ile
       50                  55                  60
   cag agc atg cac atc gac gcc acc ctg tac aca gag agc gac gtg cac      240
   Gln Ser Met His Ile Asp Ala Thr Leu Tyr Thr Glu Ser Asp Val His
   65                  70                  75                  80
   cct agc tgt aag gtg acc gcc atg aag tgc ttc ctg ctg gag ctg cag      288
   Pro Ser Cys Lys Val Thr Ala Met Lys Cys Phe Leu Leu Glu Leu Gln
                   85                  90                  95
   gtg atc agc ctg gag agc ggc gat gcc agc atc cac gac acc gtg gag      336
   Val Ile Ser Leu Glu Ser Gly Asp Ala Ser Ile His Asp Thr Val Glu
               100                 105                 110
   aac ctg atc atc ctg gcc aac aac agc ctg agc agc aac ggc aat gtg      384
   Asn Leu Ile Ile Leu Ala Asn Asn Ser Leu Ser Ser Asn Gly Asn Val
           115                 120                 125
   acc gag agc ggc tgc aag gag tgt gag gag ctg gag gag aag aac atc      432
   Thr Glu Ser Gly Cys Lys Glu Cys Glu Glu Leu Glu Glu Lys Asn Ile
       130                 135                 140
   aag gag ttc ctg cag agc ttc gtg cac atc gtg cag atg ttc atc aac      480
   Lys Glu Phe Leu Gln Ser Phe Val His Ile Val Gln Met Phe Ile Asn
   145                 150                 155                 160
   acc agc tag                                                          489
   Thr Ser
<210> 53
   <211> 162
   <212> PRT
   <213> Künstliche Sequenz
   <223> humanes IL-15-21CpG
<400> 53
   Met Arg Ile Ser Lys Pro His Leu Arg Ser Ile Ser Ile Gln Cys Tyr
   1 5 10 15
   Leu Cys Leu Leu Leu Asn Ser His Phe Leu Thr Glu Ala Gly Ile His
   20 25 30
   Val Phe Ile Leu Gly Cys Phe Ser Ala Gly Leu Pro Lys Thr Glu Ala
   35 40 45
   Asn Trp Val Asn Val Ile Ser Asp Leu Lys Lys Ile Glu Asp Leu Ile
   50 55 60
   Gln Ser Met His Ile Asp Ala Thr Leu Tyr Thr Glu Ser Asp Val His
   65 70 75 80
   Pro Ser Cys Lys Val Thr Ala Met Lys Cys Phe Leu Leu Glu Leu Gln
   85 90 95
   Val Ile Ser Leu Glu Ser Gly Asp Ala Ser Ile His Asp Thr Val Glu
   100 105 110
   Asn Leu Ile Ile Leu Ala Asn Asn Ser Leu Ser Ser Asn Gly Asn Val
   115 120 125
   Thr Glu Ser Gly Cys Lys Glu Cys Glu Glu Leu Glu Glu Lys Asn Ile
   130 135 140
   Lys Glu Phe Leu Gln Ser Phe Val His Ile Val Gln Met Phe Ile Asn
   145 150 155 160
   Thr Ser
<210> 54
   <211> 426
   <212> DNA
   <213> Künstliche Sequenz
<220>
   <223> Beschreibung der künstlichen Sequenz:synthetisches
   Gen
<220>
   <223> murines GM-CSF-62CpG
<220>
   <221> CDS
   <222> (1)..(426)
<400> 54
   atg tgg ctg cag aac ctg ctg ttc ctc ggc atc gtc gtg tac tcg ctg   48
   Met Trp Leu Gln Asn Leu Leu Phe Leu Gly Ile Val Val Tyr Ser Leu
    1               5                  10                  15
   agc gcg ccg acg cgc tcg ccg atc acc gtg acg cgg ccg tgg aag cac   96
   Ser Ala Pro Thr Arg Ser Pro Ile Thr Val Thr Arg Pro Trp Lys His
               20                  25                  30
   gtc gag gcg atc aag gag gcg ctg aac ctg ctc gac gac atg ccc gtg   144
   Val Glu Ala Ile Lys Glu Ala Leu Asn Leu Leu Asp Asp Met Pro Val
           35                  40                  45
   acg ctg aac gag gag gtc gag gtc gtg tcg aac gag ttc tcg ttc aag   192
   Thr Leu Asn Glu Glu Val Glu Val Val Ser Asn Glu Phe Ser Phe Lys
       50                  55                  60
   aag ctg acg tgc gtg cag acg cgg ctg aag atc ttc gag cag ggc ctg   240
   Lys Leu Thr Cys Val Gln Thr Arg Leu Lys Ile Phe Glu Gln Gly Leu
   65                  70                  75                  80
   cgc ggc aac ttc acg aag ctg aag ggc gcg ctg aac atg acc gcg tcg   288
   Arg Gly Asn Phe Thr Lys Leu Lys Gly Ala Leu Asn Met Thr Ala Ser
                   85                  90                  95
   tac tac cag acg tac tgc ccg ccg acg ccc gag acc gat tgc gag acg   336
   Tyr Tyr Gln Thr Tyr Cys Pro Pro Thr Pro Glu Thr Asp Cys Glu Thr
               100                 105                 110
   cag gtg acg acg tac gcc gac ttc atc gac tcg ctg aag acg ttc ctg   384
   Gln Val Thr Thr Tyr Ala Asp Phe Ile Asp Ser Leu Lys Thr Phe Leu
           115                 120                 125
   acc gac atc ccg ttc gag tgc aag aag ccc ggc cag aag tag           426
   Thr Asp Ile Pro Phe Glu Cys Lys Lys Pro Gly Gln Lys
       130                 135                 140
<210> 55
   <211> 141
   <212> PRT
   <213> Künstliche Sequenz
   <223> murines GM-CSF-62CpG
<400> 55
   Met Trp Leu Gln Asn Leu Leu Phe Leu Gly Ile Val Val Tyr Ser Leu
   1               5                   10                  15
   Ser Ala Pro Thr Arg Ser Pro Ile Thr Val Thr Arg Pro Trp Lys His
               20                  25                  30
   Val Glu Ala Ile Lys Glu Ala Leu Asn Leu Leu Asp Asp Met Pro Val
           35                  40                  45
   Thr Leu Asn Glu Glu Val Glu Val Val Ser Asn Glu Phe Ser Phe Lys
       50                  55                  60
   Lys Leu Thr Cys Val Gln Thr Arg Leu Lys Ile Phe Glu Gln Gly Leu
   65                  70                  75                  80
   Arg Gly Asn Phe Thr Lys Leu Lys Gly Ala Leu Asn Met Thr Ala Ser
                   85                  90                  95
   Tyr Tyr Gln Thr Tyr Cys Pro Pro Thr Pro Glu Thr Asp Cys Glu Thr
               100                 105                 110
   Gln Val Thr Thr Tyr Ala Asp Phe Ile Asp Ser Leu Lys Thr Phe Leu
           115                 120                 125
   Thr Asp Ile Pro Phe Glu Cys Lys Lys Pro Gly Gln Lys
       130                 135                 140
<210> 56
   <211> 489
   <212> DNA
   <213> human
<220>
   <223> humanes IL-15-wildtyp (3CpG)
<220>
   <221> CDS
   <222> (1)..(489)
<400> 56
   atg aga att tcg aaa cca cat ttg aga agt att tcc atc cag tgc tac     48
   Met Arg Ile Ser Lys Pro His Leu Arg Ser Ile Ser Ile Gln Cys Tyr
    1               5                  10                  15
   ttg tgt tta ctt cta aac agt cat ttt cta act gaa gct ggc att cat     96
   Leu Cys Leu Leu Leu Asn Ser His Phe Leu Thr Glu Ala Gly Ile His
               20                  25                  30
   gtc ttc att ttg ggc tgt ttc agt gca ggg ctt cct aaa aca gaa gcc    144
   Val Phe Ile Leu Gly Cys Phe Ser Ala Gly Leu Pro Lys Thr Glu Ala
           35                  40                  45
   aac tgg gtg aat gta ata agt gat ttg aaa aaa att gaa gat ctt att    192
   Asn Trp Val Asn Val Ile Ser Asp Leu Lys Lys Ile Glu Asp Leu Ile
       50                  55                  60
   caa tct atg cat att gat gct act tta tat acg gaa agt gat gtt cac    240
   Gln Ser Met His Ile Asp Ala Thr Leu Tyr Thr Glu Ser Asp Val His
   65                  70                  75                  80
   ccc agt tgc aaa gta aca gca atg aag tgc ttt ctc ttg gag tta caa    288
   Pro Ser Cys Lys Val Thr Ala Met Lys Cys Phe Leu Leu Glu Leu Gln
                   85                  90                  95
   gtt att tca ctt gag tcc gga gat gca agt att cat gat aca gta gaa    336
   Val Ile Ser Leu Glu Ser Gly Asp Ala Ser Ile His Asp Thr Val Glu
               100                 105                 110
   aat ctg atc atc cta gca aac aac agt ttg tct tct aat ggg aat gta    384
   Asn Leu Ile Ile Leu Ala Asn Asn Ser Leu Ser Ser Asn Gly Asn Val
           115                 120                 125
   aca gaa tct gga tgc aaa gaa tgt gag gaa ctg gag gaa aaa aat att    432
   Thr Glu Ser Gly Cys Lys Glu Cys Glu Glu Leu Glu Glu Lys Asn Ile
       130                 135                 140
   aaa gaa ttt ttg cag agt ttt gta cat att gtc caa atg ttc atc aac    480
   Lys Glu Phe Leu Gln Ser Phe Val His Ile Val Gln Met Phe Ile Asn
   145                 150                 155                 160
   act tct tag                                                        489
   Thr Ser
<210> 57
   <211> 162
   <212> PRT
   <213> human
   <223> humanes IL-15-wildtyp (3CpG)
<400> 57
   Met Arg Ile Ser Lys Pro His Leu Arg Ser Ile Ser Ile Gln Cys Tyr
    1               5                  10                  15
   Leu Cys Leu Leu Leu Asn Ser His Phe Leu Thr Glu Ala Gly Ile His
               20                  25                  30
   Val Phe Ile Leu Gly Cys Phe Ser Ala Gly Leu Pro Lys Thr Glu Ala
           35                  40                  45
   Asn Trp Val Asn Val Ile Ser Asp Leu Lys Lys Ile Glu Asp Leu Ile
        50                  55                  60
   Gln Ser Met His Ile Asp Ala Thr Leu Tyr Thr Glu Ser Asp Val His
    65                  70                  75                  80
   Pro Ser Cys Lys Val Thr Ala Met Lys Cys Phe Leu Leu Glu Leu Gln
                    85                  90                  95
   Val Ile Ser Leu Glu Ser Gly Asp Ala Ser Ile His Asp Thr Val Glu
               100                 105                 110
   Asn Leu Ile Ile Leu Ala Asn Asn Ser Leu Ser Ser Asn Gly Asn Val
           115                 120                 125
   Thr Glu Ser Gly Cys Lys Glu Cys Glu Glu Leu Glu Glu Lys Asn Ile
       130                 135                 140
   Lys Glu Phe Leu Gln Ser Phe Val His Ile Val Gln Met Phe Ile Asn
   145                 150                 155                 160
   Thr Ser
<210> 58
   <211> 426
   <212> DNA
   <213> murin
<220>
   <223> murines GM-CSF-wildtyp (11 CpG)
<220>
   <221> CDS
   <222> (1)..(426)
<400> 58
   atg tgg ctg cag aat tta ctt ttc ctg ggc att gtg gtc tac agc ctc   48
   Met Trp Leu Gln Asn Leu Leu Phe Leu Gly Ile Val Val Tyr Ser Leu
    1               5                  10                  15
   tca gca ccc acc cgc tca ccc atc act gtc acc cgg cct tgg aag cat   96
   Ser Ala Pro Thr Arg Ser Pro Ile Thr Val Thr Arg Pro Trp Lys His
               20                  25                  30
   gta gag gcc atc aaa gaa gcc ctg aac ctc ctg gat gac atg cct gtc   144
   Val Glu Ala Ile Lys Glu Ala Leu Asn Leu Leu Asp Asp Met Pro Val
           35                  40                  45
   aca ttg aat gaa gag gta gaa gtc gtc tct aac gag ttc tcc ttc aag   192
   Thr Leu Asn Glu Glu Val Glu Val Val Ser Asn Glu Phe Ser Phe Lys
       50                  55                  60
   aag cta aca tgt gtg cag acc cgc ctg aag ata ttc gag cag ggt cta   240
   Lys Leu Thr Cys Val Gln Thr Arg Leu Lys Ile Phe Glu Gln Gly Leu
   65                  70                  75                  80
   cgg ggc aat ttc acc aaa ctc aag ggc gcc ttg aac atg aca gcc agc   288
   Arg Gly Asn Phe Thr Lys Leu Lys Gly Ala Leu Asn Met Thr Ala Ser
                   85                  90                  95
   tac tac cag aca tac tgc ccc cca act ccg gaa acg gac tgt gaa aca   336
   Tyr Tyr Gln Thr Tyr Cys Pro Pro Thr Pro Glu Thr Asp Cys Glu Thr
               100                 105                 110
   caa gtt acc acc tat gcg gat ttc ata gac agc ctt aaa acc ttt ctg   384
   Gln Val Thr Thr Tyr Ala Asp Phe Ile Asp Ser Leu Lys Thr Phe Leu
           115                 120                 125
   act gat atc ccc ttt gaa tgc aaa aaa cca ggc caa aaa tag           426
   Thr Asp Ile Pro Phe Glu Cys Lys Lys Pro Gly Gln Lys
       130                 135                 140
<210> 59
   <211> 141
   <212> PRT
   <213> murin
   <223> murines GM-CSF-wildtyp (11 CpG)
<400> 59
   Met Trp Leu Gln Asn Leu Leu Phe Leu Gly Ile Val Val Tyr Ser Leu
   1 5 10 15
   Ser Ala Pro Thr Arg Ser Pro Ile Thr Val Thr Arg Pro Trp Lys His
   20 25 30
   Val Glu Ala Ile Lys Glu Ala Leu Asn Leu Leu Asp Asp Met Pro Val
   35 40 45
   Thr Leu Asn Glu Glu Val Glu Val Val Ser Asn Glu Phe Ser Phe Lys
   50 55 60
   Lys Leu Thr Cys Val Gln Thr Arg Leu Lys Ile Phe Glu Gln Gly Leu
   65 70 75 80
   Arg Gly Asn Phe Thr Lys Leu Lys Gly Ala Leu Asn Met Thr Ala Ser
   85 90 95
   Tyr Tyr Gln Thr Tyr Cys Pro Pro Thr Pro Glu Thr Asp Cys Glu Thr
   100 105 110
   Gln Val Thr Thr Tyr Ala Asp Phe Ile Asp Ser Leu Lys Thr Phe Leu
   115 120 125
   Thr Asp Ile Pro Phe Glu Cys Lys Lys Pro Gly Gln Lys
   130 135 140